# EUROPEAN PATENT APPLICATION

(11) **EP 3 851 117 A1**
(43) Date of publication of application: **21.07.2021**
(21) Application number: 20382026.1
(22) Date of filing: 20.01.2020
(51) Int. Cl.: A61K 38/16, A61K 45/00, A61P 31/04, G01N 33/48

(54) **INHIBITORS OF INTRACELLULAR BACTERIAL GROWTH AND PERSISTENCE AS ANTIBIOTICS**

(71) Applicant: Consejo Superior de Investigaciones Científicas (CSIC), 28006 Madrid (ES); Universidad Autónoma De Madrid (UAM), 28049 Madrid (ES)
(72) Inventor: GARCÍA DEL PORTILLO, Francisco, 28049 Madrid (ES); CASTANHEIRA, Sónia, 28049 Madrid (ES); CESTERO CARRILLO, Juan José, 28049 Madrid (ES); LÓPEZ ESCARPA, David, 28049 Madrid (ES); PUCCIARELLI MORRONE, María Graciela, 28049 Madrid (ES)
(74) Representative: Pons

(57) **Abstract**

The invention relates to an *in vitro* screening method to identify inhibitors of intracellular bacterial growth and persistence and uses thereof as medicament, preferably as antibiotics. Furthermore, the present invention also relates to the inhibitors for use in the treatment and/or prevention of intracellular bacterial infections.

SEQUENCE LISTING

<110>  Consejo Superior de Investigaciones Científicas (CSIC)
       Universidad Autónoma de Madrid (UAM)

<120>  Inhibitors of intracellular bacterial growth and persistence as
       antibiotics

<130>  EP1641.1498

<160>  42

<170>  PatentIn version 3.5

<210>  1
<211>  1872
<212>  DNA
<213>  Salmonella typhimurium

<400>  1
atgactttta aagactttga tgcggaagag aaactcttcc tgagacgtgt gattgtggcg      60

tttggtgtag tggttgtctg ctttggtatc ctgatcttca acctctataa cttgcaaatc     120

cgccagcacc actactacac cacccgttcg aatgagaacg atatcaagat gctacccgtc     180

gcgcccacgc gcggcatcat ttacgatcgc aacggcattc cgctggtgcg taacgtaacc     240

tggtatgata tcgcggttac gccctataaa attgccgata tggatgcgct gctaaaacag     300

ctcaccccta ttgttgatct ctcccccgac gatatctccg actttcgccg tgcgctaaaa     360

tccagtagcc gctatcgtcc ggtggtgctc aaaaacgcgc taacggatgt tgaaatcgca     420

cgctttgcgg tcaatcagtt tcatttcaac ggcgtcacga tcaatagcta tcaggatcgg     480

cagtatcctt acggcgcgga gctggcgcat gttctcggtt acgtgtcaaa gataaacgat     540

aacgacctta aagcgctgga taaaaaaggg ctggcagaga attacgcggc ggaccataac     600

atcggtaaac aggggattga gcgttactac gaaaacgatc ttcatggtaa aaccggttat     660

caggaggtcg aggtggataa tcacgggcgt attgtccgcc tgctgaaaga cgttcctccc     720

atcgcgggta agaacattca cctgacgctg gatctccatt tacaagagta cattgaaagt     780

ttgctggccg ggcagcgtgc tgccgtactg gttgaagatc cgcacgatgg ttcagtgctg     840

gcgatggtat ctatgcccag ctacgacccg aacccctttg taaaaggcat tagctatcag     900

gattacggca aactgctgca cgataaaaat ctgccgctga tcaaccgcgt gacgcaaggg     960

ctttatcctc cggcgtcaac ggttaaaccc tatatggcaa tgtccgcgct gttatgcggg    1020

attattacgc cgcaaaccac cttctttggc gcgccaacct ggacgcttcc cggcacgcag    1080

cggcattacc gggactggaa aaaaaccgga cacggtatgc tggatgtcac taaagcgatt    1140

gaggaatctg cggatacctt tttctatcag gtcgcttata tgatgggtat tgaccgtatc    1200

gacacgatgt tgtctcagtt cggctatggc aaaccgacgg gcatcgacct taatgaagaa    1260

tatgacgggc tgcttcccag ccgcgcatgg aaacagcggg tccataaaaa agcctggtat    1320

cagggcgata ccatttctgt cggcatcggc cagggttact ggattgccac ccccatccag 1380

atggtaaaag cgatggtggc gctcatcaac aacggtaagg tgattgcccc tcacctgtta 1440

ctcaatgaag agagcggcaa aacggtggtt ccgtatcgtc cttcaggaac accggcacag 1500

atagccgatc cggcgtcgcc atactgggga ctggtgcgtc aggcgatgta tggcatggcg 1560

aatgcaccca acggaacggg ctataagttc tttcacaccg cgccctacgg cattgcggcc 1620

aaaagcggca cgtcgcaggt atttagcctg aaagaaaatc agacctacaa cgcgaaaatg 1680

atccccattc gcctgcgcga tcatgtgttt tataccgctt ttgcaccgta taaaaaccct 1740

aaggtcgcta ttgccttgat tctggaaaac ggcggaagcg acggcgttac cgccgcaccc 1800

atcatgcgaa aaatccttga ccacctgttt gatccacagg ctgataccac acagccggat 1860

caggcgccat ag 1872

<210> 2
<211> 623
<212> PRT
<213> Salmonella typhimurium

<400> 2

Met Thr Phe Lys Asp Phe Asp Ala Glu Glu Lys Leu Phe Leu Arg Arg
1               5                   10                  15

Val Ile Val Ala Phe Gly Val Val Val Val Cys Phe Gly Ile Leu Ile
            20                  25                  30

Phe Asn Leu Tyr Asn Leu Gln Ile Arg Gln His His Tyr Tyr Thr Thr
        35                  40                  45

Arg Ser Asn Glu Asn Asp Ile Lys Met Leu Pro Val Ala Pro Thr Arg
    50                  55                  60

Gly Ile Ile Tyr Asp Arg Asn Gly Ile Pro Leu Val Arg Asn Val Thr
65                  70                  75                  80

Trp Tyr Asp Ile Ala Val Thr Pro Tyr Lys Ile Ala Asp Met Asp Ala
                85                  90                  95

Leu Leu Lys Gln Leu Thr Pro Ile Val Asp Leu Ser Pro Asp Asp Ile
            100                 105                 110

Ser Asp Phe Arg Arg Ala Leu Lys Ser Ser Ser Arg Tyr Arg Pro Val
        115                 120                 125

Val Leu Lys Asn Ala Leu Thr Asp Val Glu Ile Ala Arg Phe Ala Val
    130                 135                 140

Asn Gln Phe His Phe Asn Gly Val Thr Ile Asn Ser Tyr Gln Asp Arg
145                 150                 155                 160

Gln Tyr Pro Tyr Gly Ala Glu Leu Ala His Val Leu Gly Tyr Val Ser
                165                 170                 175

Lys Ile Asn Asp Asn Asp Leu Lys Ala Leu Asp Lys Lys Gly Leu Ala
            180                 185                 190

Glu Asn Tyr Ala Ala Asp His Asn Ile Gly Lys Gln Gly Ile Glu Arg
        195                 200                 205

Tyr Tyr Glu Asn Asp Leu His Gly Lys Thr Gly Tyr Gln Glu Val Glu
    210                 215                 220

Val Asp Asn His Gly Arg Ile Val Arg Leu Leu Lys Asp Val Pro Pro
225                 230                 235                 240

Ile Ala Gly Lys Asn Ile His Leu Thr Leu Asp Leu His Leu Gln Glu
                245                 250                 255

Tyr Ile Glu Ser Leu Leu Ala Gly Gln Arg Ala Ala Val Leu Val Glu
            260                 265                 270

Asp Pro His Asp Gly Ser Val Leu Ala Met Val Ser Met Pro Ser Tyr
        275                 280                 285

Asp Pro Asn Pro Phe Val Lys Gly Ile Ser Tyr Gln Asp Tyr Gly Lys
    290                 295                 300

Leu Leu His Asp Lys Asn Leu Pro Leu Ile Asn Arg Val Thr Gln Gly
305                 310                 315                 320

Leu Tyr Pro Pro Ala Ser Thr Val Lys Pro Tyr Met Ala Met Ser Ala
                325                 330                 335

Leu Leu Cys Gly Ile Ile Thr Pro Gln Thr Thr Phe Phe Gly Ala Pro
            340                 345                 350

Thr Trp Thr Leu Pro Gly Thr Gln Arg His Tyr Arg Asp Trp Lys Lys
        355                 360                 365

Thr Gly His Gly Met Leu Asp Val Thr Lys Ala Ile Glu Glu Ser Ala
    370                 375                 380

Asp Thr Phe Phe Tyr Gln Val Ala Tyr Met Met Gly Ile Asp Arg Ile

385                 390                 395                 400

Asp Thr Met Leu Ser Gln Phe Gly Tyr Gly Lys Pro Thr Gly Ile Asp
                405                 410                 415

Leu Asn Glu Glu Tyr Asp Gly Leu Leu Pro Ser Arg Ala Trp Lys Gln
            420                 425                 430

Arg Val His Lys Lys Ala Trp Tyr Gln Gly Asp Thr Ile Ser Val Gly
        435                 440                 445

Ile Gly Gln Gly Tyr Trp Ile Ala Thr Pro Ile Gln Met Val Lys Ala
    450                 455                 460

Met Val Ala Leu Ile Asn Asn Gly Lys Val Ile Ala Pro His Leu Leu
465                 470                 475                 480

Leu Asn Glu Glu Ser Gly Lys Thr Val Val Pro Tyr Arg Pro Ser Gly
                485                 490                 495

Thr Pro Ala Gln Ile Ala Asp Pro Ala Ser Pro Tyr Trp Gly Leu Val
            500                 505                 510

Arg Gln Ala Met Tyr Gly Met Ala Asn Ala Pro Asn Gly Thr Gly Tyr
        515                 520                 525

Lys Phe Phe His Thr Ala Pro Tyr Gly Ile Ala Ala Lys Ser Gly Thr
    530                 535                 540

Ser Gln Val Phe Ser Leu Lys Glu Asn Gln Thr Tyr Asn Ala Lys Met
545                 550                 555                 560

Ile Pro Ile Arg Leu Arg Asp His Val Phe Tyr Thr Ala Phe Ala Pro
                565                 570                 575

Tyr Lys Asn Pro Lys Val Ala Ile Ala Leu Ile Leu Glu Asn Gly Gly
            580                 585                 590

Ser Asp Gly Val Thr Ala Ala Pro Ile Met Arg Lys Ile Leu Asp His
        595                 600                 605

Leu Phe Asp Pro Gln Ala Asp Thr Thr Gln Pro Asp Gln Ala Pro
    610                 615                 620

<210>  3
<211>  1746
<212>  DNA
<213>  Salmonella typhimurium

<400> 3
gtgaaaaaga aaagcgacgg cgatacccgt aattttacgc ccatacgttt tgcactactt 60

tgcactgcca ttctgctcag tatggcattg ttgttagggc gggtggcatg gctgcaaatt 120

gttacgccgt cgaaactggt gaaacaagaa gatatgcgct cgcttcgtga agtgaccacg 180

gcctctccac gcggcatgat caccgacaga gaagggcgac ccttagcggt gagcgttccg 240

gttaatgctg tctgggccga tccgaagact atcataagca aaggcggcgt tggctacaat 300

gagcgctggc aggcgctggc cagcgccctg catctgtctc tcagtacgct tgcggaacgg 360

gtaaacagta acccggcagg ccgttttatc taccttgcgc gccaggtttc accccagcaa 420

gccgaatgga tagataaact gaatctaccg ggtattaacc tgcgggaaga gtcgcgccgt 480

ttttaccctg ccggacacgt agcggccaat ttgatcggct ttaccaatat cgacgggcag 540

ggaatagaag gtattgaaaa gagctttaac gcgcaactga cgggaaaacc gggctcccga 600

ttggtgcgta aagataaatt tggtcacgtt attgagaaca ttacggaagt gaatccggtt 660

cctgctcatg aacttcagct tagcattgat gaacgtctgc aaacggttac cgaagatgcg 720

ctggataacg ccgtcatctg gaacaaagcg gaatccggcg ctgccgtatt gattaacatc 780

ccaacgggtg aaattctctc gatggcgagc tacccggatt tcaacccgaa caaccgtgaa 840

ggcgcgcagt tggacgattt tcgtaaccgc gcgattagcg acaccttcga acccggttct 900

actgtgaagc cattggtcat tatgactgcg ttacagcagg gcatcgtgca gccggacagc 960

gttatcgata cccatccttt tactctcgat ggacaccgta tccgcgacgt cggttactat 1020

ccggaactga cattaaccgg cattttgcaa aaatccagcg ataccggcgt ttctcacctt 1080

tctctggcga tgccggtgca gaaattaatg gatacttata aaagctttgg tttcggcgtg 1140

cctaccggac tgggtctcac cggagagagc agcgggttat taccgaaacg ccgttactgg 1200

agtgatttag atcgcgctac ctttgctttt ggctatggat taatggtgac gccgctgcaa 1260

ctggcgcatg tttatgccac gattggcagc tttggtatct accgtccgtt atcgatcacc 1320

aaagttgatc cgcccgtgat cggcacgcgg gtaatgccgg aagaactggt gcacgaggtg 1380

gaacatatga tggagagcgt cgcgttgcct ggcggcggcg gaacaaaagc cgcagtaaga 1440

gactaccgta ttgcggtcaa aaccgggacg gcgaaaaaaa ttggcgatga tggtaaatac 1500

gtcgataagt atgtggcata taccgcaggc gtcgcgccgg caagtaaccc cagatttgcg 1560

ctggtggtgg tgattaataa tccgcaaaat ggggcttatt atggcggtgc ggtatctgcg 1620

cccgtattca gccagatcat gggcgatgtg ctgcggctgg aaaatgttga gccagacggt 1680

atgccagccg attcagatca ccttctggtg atgcatggca gccacgttgc ggttccgggt 1740

tcgtaa 1746

<210>  4
<211>  581
<212>  PRT
<213>  Salmonella typhimurium

<400>  4

Met Lys Lys Lys Ser Asp Gly Asp Thr Arg Asn Phe Thr Pro Ile Arg
1               5                   10                  15

Phe Ala Leu Leu Cys Thr Ala Ile Leu Leu Ser Met Ala Leu Leu Leu
            20                  25                  30

Gly Arg Val Ala Trp Leu Gln Ile Val Thr Pro Ser Lys Leu Val Lys
        35                  40                  45

Gln Glu Asp Met Arg Ser Leu Arg Glu Val Thr Thr Ala Ser Pro Arg
    50                  55                  60

Gly Met Ile Thr Asp Arg Glu Gly Arg Pro Leu Ala Val Ser Val Pro
65                  70                  75                  80

Val Asn Ala Val Trp Ala Asp Pro Lys Thr Ile Ile Ser Lys Gly Gly
                85                  90                  95

Val Gly Tyr Asn Glu Arg Trp Gln Ala Leu Ala Ser Ala Leu His Leu
            100                 105                 110

Ser Leu Ser Thr Leu Ala Glu Arg Val Asn Ser Asn Pro Ala Gly Arg
        115                 120                 125

Phe Ile Tyr Leu Ala Arg Gln Val Ser Pro Gln Gln Ala Glu Trp Ile
    130                 135                 140

Asp Lys Leu Asn Leu Pro Gly Ile Asn Leu Arg Glu Glu Ser Arg Arg
145                 150                 155                 160

Phe Tyr Pro Ala Gly His Val Ala Ala Asn Leu Ile Gly Phe Thr Asn
                165                 170                 175

Ile Asp Gly Gln Gly Ile Glu Gly Ile Glu Lys Ser Phe Asn Ala Gln
            180                 185                 190

Leu Thr Gly Lys Pro Gly Ser Arg Leu Val Arg Lys Asp Lys Phe Gly
        195                 200                 205

His Val Ile Glu Asn Ile Thr Glu Val Asn Pro Val Pro Ala His Glu
    210                 215                 220

Leu Gln Leu Ser Ile Asp Glu Arg Leu Gln Thr Val Thr Glu Asp Ala
225                 230                 235                 240

Leu Asp Asn Ala Val Ile Trp Asn Lys Ala Glu Ser Gly Ala Ala Val
                245                 250                 255

Leu Ile Asn Ile Pro Thr Gly Glu Ile Leu Ser Met Ala Ser Tyr Pro
            260                 265                 270

Asp Phe Asn Pro Asn Asn Arg Glu Gly Ala Gln Leu Asp Asp Phe Arg
        275                 280                 285

Asn Arg Ala Ile Ser Asp Thr Phe Glu Pro Gly Ser Thr Val Lys Pro
    290                 295                 300

Leu Val Ile Met Thr Ala Leu Gln Gln Gly Ile Val Gln Pro Asp Ser
305                 310                 315                 320

Val Ile Asp Thr His Pro Phe Thr Leu Asp Gly His Arg Ile Arg Asp
                325                 330                 335

Val Gly Tyr Tyr Pro Glu Leu Thr Leu Thr Gly Ile Leu Gln Lys Ser
            340                 345                 350

Ser Asp Thr Gly Val Ser His Leu Ser Leu Ala Met Pro Val Gln Lys
        355                 360                 365

Leu Met Asp Thr Tyr Lys Ser Phe Gly Phe Gly Val Pro Thr Gly Leu
    370                 375                 380

Gly Leu Thr Gly Glu Ser Ser Gly Leu Leu Pro Lys Arg Arg Tyr Trp
385                 390                 395                 400

Ser Asp Leu Asp Arg Ala Thr Phe Ala Phe Gly Tyr Gly Leu Met Val
                405                 410                 415

Thr Pro Leu Gln Leu Ala His Val Tyr Ala Thr Ile Gly Ser Phe Gly
            420                 425                 430

Ile Tyr Arg Pro Leu Ser Ile Thr Lys Val Asp Pro Pro Val Ile Gly
        435                 440                 445

Thr Arg Val Met Pro Glu Glu Leu Val His Glu Val Glu His Met Met
    450                 455                 460

Glu Ser Val Ala Leu Pro Gly Gly Gly Gly Thr Lys Ala Ala Val Arg
465                 470                 475                 480

Asp Tyr Arg Ile Ala Val Lys Thr Gly Thr Ala Lys Lys Ile Gly Asp
                485                 490                 495

Asp Gly Lys Tyr Val Asp Lys Tyr Val Ala Tyr Thr Ala Gly Val Ala
            500                 505                 510

Pro Ala Ser Asn Pro Arg Phe Ala Leu Val Val Val Ile Asn Asn Pro
        515                 520                 525

Gln Asn Gly Ala Tyr Tyr Gly Gly Ala Val Ser Ala Pro Val Phe Ser
    530                 535                 540

Gln Ile Met Gly Asp Val Leu Arg Leu Glu Asn Val Glu Pro Asp Gly
545                 550                 555                 560

Met Pro Ala Asp Ser Asp His Leu Leu Val Met His Gly Ser His Val
                565                 570                 575

Ala Val Pro Gly Ser
            580

<210>  5
<211>  1902
<212>  DNA
<213>  Salmonella typhimurium

<400>  5
atgaaacgac aaaattcttt tcgtgactat acggctgagt ccgcactgtt tgtgcgtcgg      60

gcgctggtcg cctttttggg tattttgctg ctgaccggcg tactgatcgc caatttgtat     120

aatctgcaaa tccttcgttt taccgattac cagacccgct cgaacgaaaa ccgtatcaag     180

ctggtgccaa ttgcgccaag ccgcgggata atctacgacc gtaacggcat tccccttgcg      240

ctgaaccgca ctatctatca gattgaaatg atgccggaaa aggtggataa cgttcagcaa     300

acgctggatg cgctacgcag tgtggtcgat ttaaacgacg acgatatcgc cgcctttaaa     360

aaagagcgcg cccgatccca tcgctttact tctattccgg tgaaaacaaa cctgacggaa     420

gttcaggtcg cgcgctttgc tgtcaaccag taccgttttc cgggcgtcga agtcaaaggc     480

tataaacgcc gctattatcc atacggatcg gcgctgaccc acgttatcgg ctacgtatcg     540

aaaatcaatg ataaagacgt cgagcgtctc gacagagaaa acaaactggc gaactacgcc     600

gccacccatg acatcggcaa gctgggcatt gagcgctact atgaagatat tctgcatggg     660

caaaccggtt atgaagaggt tgaagtcaac aaccgcggtc gcgttattcg ccaactcaaa     720

gaggtgccgc cgcaggccgg acacgatatc tacctgacgc tggatctcaa gctccagcag     780

tatattgaaa cgctgctggc aggcagtcgc gcggcggtga ttgtcaccga tccacgtacc     840

ggcggcgtgc tatcgctggt ctccatgcca agttacgacc ctaacctttt cgtggatggc 900

atctccagca aagattattc cgggctgctt aacgacccga acacgccatt agtgaaccgc 960

gcgactcagg gtgtctaccc acctgcttct accgttaagc cttacgtcgc cgtatcggcg 1020

ttaagcgctg gcgtcatcac ccgtaacacc agtcttttcg accctggctg gtggcagttg 1080

ccggggtctg agaaacgcta ccgcgactgg aaaaaatggg gccacgggca tctgaatatc 1140

accaaatccc ttgaagagtc ggcggatacc ttcttctatc aggttgccta tgatatgggg 1200

atcgatcgcc tgtcagaatg gatgggtaag ttcggctatg gtcactacac cggaattgat 1260

ctcgcagaag agcgctctgg taatatgccg acccgcgagt ggaaacagaa acgctttaaa 1320

aaaccgtggt atcagggcga tacgattccg gtgggcatcg gccagggtta ctggacagcg 1380

acgccgattc agatgagtaa ggcgctaatg atccttatta acgacggtgt agttaaagtc 1440

ccgcacctgt taatgagcac cgcggaaaac ggtaaacagg tgccgtgggt acagccgcat 1500

gagccgccgg tgggcgatat tcactccggc tactgggaaa ttgcgaaaga tggcatgtat 1560

ggcgtcgcca accgccctaa cggcacggcg cataaatatt ttgccagcgc gccctataaa 1620

atcgcggcga agtcgggcac cgcgcaggtc tttggcctga aggccaacga aacgtacaat 1680

gcgcataaaa tcgccgagcg cctgcgcgac cataaattga tgaccgcctt tgcgccgtat 1740

aacaatcctc aggtcgccgt cgcgatcatc cttgaaaacg gcggcgcagg cccggcagta 1800

ggcacgatca tgcgccaaat ccttgatcac atcatgctgg gcgacaacaa cacccatttg 1860

cctgcggaaa acccggtggt tgcagcggcg gaggaccaat aa 1902

<210> 6
<211> 633
<212> PRT
<213> Salmonella typhimurium

<400> 6

Met Lys Arg Gln Asn Ser Phe Arg Asp Tyr Thr Ala Glu Ser Ala Leu
1               5                   10                  15

Phe Val Arg Arg Ala Leu Val Ala Phe Leu Gly Ile Leu Leu Leu Thr
            20                  25                  30

Gly Val Leu Ile Ala Asn Leu Tyr Asn Leu Gln Ile Leu Arg Phe Thr
        35                  40                  45

Asp Tyr Gln Thr Arg Ser Asn Glu Asn Arg Ile Lys Leu Val Pro Ile
    50                  55                  60

Ala Pro Ser Arg Gly Ile Ile Tyr Asp Arg Asn Gly Ile Pro Leu Ala
65                  70                  75                  80

Leu Asn Arg Thr Ile Tyr Gln Ile Glu Met Met Pro Glu Lys Val Asp
                85                  90                  95

Asn Val Gln Gln Thr Leu Asp Ala Leu Arg Ser Val Val Asp Leu Asn
            100                 105                 110

Asp Asp Asp Ile Ala Ala Phe Lys Lys Glu Arg Ala Arg Ser His Arg
        115                 120                 125

Phe Thr Ser Ile Pro Val Lys Thr Asn Leu Thr Glu Val Gln Val Ala
    130                 135                 140

Arg Phe Ala Val Asn Gln Tyr Arg Phe Pro Gly Val Glu Val Lys Gly
145                 150                 155                 160

Tyr Lys Arg Arg Tyr Tyr Pro Tyr Gly Ser Ala Leu Thr His Val Ile
                165                 170                 175

Gly Tyr Val Ser Lys Ile Asn Asp Lys Asp Val Glu Arg Leu Asp Arg
            180                 185                 190

Glu Asn Lys Leu Ala Asn Tyr Ala Ala Thr His Asp Ile Gly Lys Leu
        195                 200                 205

Gly Ile Glu Arg Tyr Tyr Glu Asp Ile Leu His Gly Gln Thr Gly Tyr
    210                 215                 220

Glu Glu Val Glu Val Asn Asn Arg Gly Arg Val Ile Arg Gln Leu Lys
225                 230                 235                 240

Glu Val Pro Pro Gln Ala Gly His Asp Ile Tyr Leu Thr Leu Asp Leu
                245                 250                 255

Lys Leu Gln Gln Tyr Ile Glu Thr Leu Leu Ala Gly Ser Arg Ala Ala
            260                 265                 270

Val Ile Val Thr Asp Pro Arg Thr Gly Gly Val Leu Ser Leu Val Ser
        275                 280                 285

Met Pro Ser Tyr Asp Pro Asn Leu Phe Val Asp Gly Ile Ser Ser Lys
    290                 295                 300

Asp Tyr Ser Gly Leu Leu Asn Asp Pro Asn Thr Pro Leu Val Asn Arg
305                 310                 315                 320

Ala Thr Gln Gly Val Tyr Pro Pro Ala Ser Thr Val Lys Pro Tyr Val
                325                 330                 335

Ala Val Ser Ala Leu Ser Ala Gly Val Ile Thr Arg Asn Thr Ser Leu
        340                 345                 350

Phe Asp Pro Gly Trp Trp Gln Leu Pro Gly Ser Glu Lys Arg Tyr Arg
        355                 360                 365

Asp Trp Lys Lys Trp Gly His Gly His Leu Asn Ile Thr Lys Ser Leu
    370                 375                 380

Glu Glu Ser Ala Asp Thr Phe Phe Tyr Gln Val Ala Tyr Asp Met Gly
385                 390                 395                 400

Ile Asp Arg Leu Ser Glu Trp Met Gly Lys Phe Gly Tyr Gly His Tyr
                405                 410                 415

Thr Gly Ile Asp Leu Ala Glu Glu Arg Ser Gly Asn Met Pro Thr Arg
            420                 425                 430

Glu Trp Lys Gln Lys Arg Phe Lys Lys Pro Trp Tyr Gln Gly Asp Thr
        435                 440                 445

Ile Pro Val Gly Ile Gly Gln Gly Tyr Trp Thr Ala Thr Pro Ile Gln
    450                 455                 460

Met Ser Lys Ala Leu Met Ile Leu Ile Asn Asp Gly Val Val Lys Val
465                 470                 475                 480

Pro His Leu Leu Met Ser Thr Ala Glu Asn Gly Lys Gln Val Pro Trp
                485                 490                 495

Val Gln Pro His Glu Pro Pro Val Gly Asp Ile His Ser Gly Tyr Trp
            500                 505                 510

Glu Ile Ala Lys Asp Gly Met Tyr Gly Val Ala Asn Arg Pro Asn Gly
        515                 520                 525

Thr Ala His Lys Tyr Phe Ala Ser Ala Pro Tyr Lys Ile Ala Ala Lys
    530                 535                 540

Ser Gly Thr Ala Gln Val Phe Gly Leu Lys Ala Asn Glu Thr Tyr Asn
545                 550                 555                 560

Ala His Lys Ile Ala Glu Arg Leu Arg Asp His Lys Leu Met Thr Ala
                565                 570                 575

Phe Ala Pro Tyr Asn Asn Pro Gln Val Ala Val Ala Ile Ile Leu Glu

580                 585                 590

Asn Gly Gly Ala Gly Pro Ala Val Gly Thr Ile Met Arg Gln Ile Leu
        595                 600                 605

Asp His Ile Met Leu Gly Asp Asn Asn Thr His Leu Pro Ala Glu Asn
    610                 615                 620

Pro Val Val Ala Ala Ala Glu Asp Gln
625                 630

<210>  7
<211>  1767
<212>  DNA
<213>  Salmonella typhimurium

<400>  7
atgaaagcag cggcaaaaac gcaaaaatcg aaacgccagg aagaacagac caacttcatc      60

agttggcgtt ttgcgttgct gtgcggctgt attttactgg cgctggtttt tttacttgga     120

cgcgcggcgt ggctacagat tatcgccccg gatatgctgg tgcgtcaggg cgatatgcgt     180

tcgctgcgtg ttcaggaagt ctccacctca cgcggtatga ttaccgaccg ttccgggcgt     240

ccactggcgg tcagcgtacc ggttaaggcg atctgggccg acccgaaaga agttcatgac     300

gccggcggca tcagcgttgg cgatcgctgg aaagcgcttt ctactgcgct caatattcca     360

ctcgatcagc tctccgcccg tatcaacgcc aacccgaaag ggcgttttat ttatctggcg     420

cgccaggtaa atcctgacat ggcggactac atcaaaaaac tcaaactgcc ggggattcat     480

ttgcgtgaag agtcccgccg ctactacccg tcaggagaag taaccgctca cctcatcggt     540

tttaccaacg tcgacagtca ggggattgaa ggcgttgaaa agagctttga taagtggctt     600

accggtcagc ctggcgagcg aatcgtgcgt aaagaccgct atggccgcgt gattgaggat     660

atctcctcca ctgacagtca ggcagcgcac aatctggcgt taagcattga tgaacgctta     720

caggcgctgg tttaccgcga actgaacaac gcggtggcgt ttaacaaggc ggaatccggt     780

agcgcggtgc tggtcgatgt caataccggt gaagtactgg cgatggcgaa tagcccttcc     840

tataacccca acaaccttgc cggtacgccg aaagacgcga tgcgtaaccg taccatcacc     900

gacgtgtttg aaccgggttc tacggttaag ccgatggtgg tgatgactgc gcttcagcgc     960

ggtatcgtca acgaaaatac ggtgttgaat accgttccat accgaattaa cggccacgaa    1020

attaaagacg tggcgcgcta cagcgaatta accctgaccg gggttctaca gaagtcgagt    1080

aacgtcggtg tatccaaact ggcgttagcg atgccgtcct cagcgttagt agatacttac    1140

tcacgttttg ggctaggaaa ggcgaccaat ttggggttgg tcggagaacg cagtggctta    1200

tatcctcaaa aacaacggtg gtccgacata gagagggcca ccttctcttt cggctacggg    1260

ctaatggtaa cgccgttaca gttagcgcga gtctatgcaa ccatcggcag ctatggcatt 1320

tatcgcccgc tgtcgattac caaagttgat ccgcctgtac ccggcgaacg catcttccct 1380

gaatccaccg tacgcaccgt ggtgcatatg atggaaagcg tggcgctgcc gggcggcggc 1440

ggcgtgaagg cagcgattaa aggctaccgt atcgcgatta aaaccggtac ggcgaaaaaa 1500

gtggggccgg acggtcgcta catcaacaaa tacattgctt acaccgcagg cgtcgcgcct 1560

gcgagtcagc cgcgcttcgc gctggtggtt gtcatcaacg atccgcaggc gggtaaatac 1620

tacggcggcg ccgtttccgc gccggtattt ggtgccatca tgggcggcgt tctgcgcacc 1680

atgaacatcg aaccggacgc gctggcaacg ggcgaaaaaa atgaattcgt aattaatcaa 1740

ggcgagggaa caggtggcag atcgtaa 1767

<210> 8
<211> 588
<212> PRT
<213> Salmonella typhimurium

<400> 8

Met Lys Ala Ala Ala Lys Thr Gln Lys Ser Lys Arg Gln Glu Glu Gln
1               5                   10                  15

Thr Asn Phe Ile Ser Trp Arg Phe Ala Leu Leu Cys Gly Cys Ile Leu
            20                  25                  30

Leu Ala Leu Val Phe Leu Leu Gly Arg Ala Ala Trp Leu Gln Ile Ile
        35                  40                  45

Ala Pro Asp Met Leu Val Arg Gln Gly Asp Met Arg Ser Leu Arg Val
    50                  55                  60

Gln Glu Val Ser Thr Ser Arg Gly Met Ile Thr Asp Arg Ser Gly Arg
65                  70                  75                  80

Pro Leu Ala Val Ser Val Pro Val Lys Ala Ile Trp Ala Asp Pro Lys
                85                  90                  95

Glu Val His Asp Ala Gly Gly Ile Ser Val Gly Asp Arg Trp Lys Ala
            100                 105                 110

Leu Ser Thr Ala Leu Asn Ile Pro Leu Asp Gln Leu Ser Ala Arg Ile
        115                 120                 125

Asn Ala Asn Pro Lys Gly Arg Phe Ile Tyr Leu Ala Arg Gln Val Asn
    130                 135                 140

Pro Asp Met Ala Asp Tyr Ile Lys Lys Leu Lys Leu Pro Gly Ile His

145                 150                 155                 160

Leu Arg Glu Glu Ser Arg Arg Tyr Tyr Pro Ser Gly Glu Val Thr Ala
                165                 170                 175

His Leu Ile Gly Phe Thr Asn Val Asp Ser Gln Gly Ile Glu Gly Val
            180                 185                 190

Glu Lys Ser Phe Asp Lys Trp Leu Thr Gly Gln Pro Gly Glu Arg Ile
        195                 200                 205

Val Arg Lys Asp Arg Tyr Gly Arg Val Ile Glu Asp Ile Ser Ser Thr
    210                 215                 220

Asp Ser Gln Ala Ala His Asn Leu Ala Leu Ser Ile Asp Glu Arg Leu
225                 230                 235                 240

Gln Ala Leu Val Tyr Arg Glu Leu Asn Asn Ala Val Ala Phe Asn Lys
                245                 250                 255

Ala Glu Ser Gly Ser Ala Val Leu Val Asp Val Asn Thr Gly Glu Val
            260                 265                 270

Leu Ala Met Ala Asn Ser Pro Ser Tyr Asn Pro Asn Asn Leu Ala Gly
        275                 280                 285

Thr Pro Lys Asp Ala Met Arg Asn Arg Thr Ile Thr Asp Val Phe Glu
    290                 295                 300

Pro Gly Ser Thr Val Lys Pro Met Val Val Met Thr Ala Leu Gln Arg
305                 310                 315                 320

Gly Ile Val Asn Glu Asn Thr Val Leu Asn Thr Val Pro Tyr Arg Ile
                325                 330                 335

Asn Gly His Glu Ile Lys Asp Val Ala Arg Tyr Ser Glu Leu Thr Leu
            340                 345                 350

Thr Gly Val Leu Gln Lys Ser Ser Asn Val Gly Val Ser Lys Leu Ala
        355                 360                 365

Leu Ala Met Pro Ser Ser Ala Leu Val Asp Thr Tyr Ser Arg Phe Gly
    370                 375                 380

Leu Gly Lys Ala Thr Asn Leu Gly Leu Val Gly Glu Arg Ser Gly Leu
385                 390                 395                 400

Tyr Pro Gln Lys Gln Arg Trp Ser Asp Ile Glu Arg Ala Thr Phe Ser
                405                 410                 415

Phe Gly Tyr Gly Leu Met Val Thr Pro Leu Gln Leu Ala Arg Val Tyr
            420                 425                 430

Ala Thr Ile Gly Ser Tyr Gly Ile Tyr Arg Pro Leu Ser Ile Thr Lys
        435                 440                 445

Val Asp Pro Pro Val Pro Gly Glu Arg Ile Phe Pro Glu Ser Thr Val
    450                 455                 460

Arg Thr Val Val His Met Met Glu Ser Val Ala Leu Pro Gly Gly Gly
465                 470                 475                 480

Gly Val Lys Ala Ala Ile Lys Gly Tyr Arg Ile Ala Ile Lys Thr Gly
                485                 490                 495

Thr Ala Lys Lys Val Gly Pro Asp Gly Arg Tyr Ile Asn Lys Tyr Ile
            500                 505                 510

Ala Tyr Thr Ala Gly Val Ala Pro Ala Ser Gln Pro Arg Phe Ala Leu
        515                 520                 525

Val Val Val Ile Asn Asp Pro Gln Ala Gly Lys Tyr Tyr Gly Gly Ala
    530                 535                 540

Val Ser Ala Pro Val Phe Gly Ala Ile Met Gly Gly Val Leu Arg Thr
545                 550                 555                 560

Met Asn Ile Glu Pro Asp Ala Leu Ala Thr Gly Glu Lys Asn Glu Phe
                565                 570                 575

Val Ile Asn Gln Gly Glu Gly Thr Gly Gly Arg Ser
            580                 585

<210>  9
<211>  1746
<212>  DNA
<213>  Citrobacter freundii

<400>  9
gtgaaaaaga aaaacgacgg cgacgcgcgc aattttactc ccgtacgttt tgccctgctt      60

tgtgtggcaa tattattgag tctgggattg ctgctgggtc gggttgcatg gctgcaaatt     120

gtcacgccga cgagtctggt gaaacaggaa gacatgcgtt cattacgtga ggtaactatt     180

gcctcgccgc gcgggatgat taccgacaga gaagggcgcc cgctggcggt aagtgttccg     240

gttaacgccg tatgggccga tcctaagacc atcatcagca agggaggcgt gggttataac     300

gaacggtggc aagctctggc aagggctctg catctgtcac tgagtacgct ggcggaacgc 360

gttaacagta atcctggcgg acgttttatt tacctggcac gacaaatttc ccctcagcag 420

gctgagtggg tcgatgatct taatctcccg ggcattaacc tgcgtgagga atcccgccgg 480

ttctatcctg caggccacgt agcggcgaat ctcataggtt ttaccaacat tgatggacaa 540

ggcattgaag ggctggaaaa aagctttaat aaccagttga tgggtaaacc tggctcccgt 600

ctggtacgta aagacaaatt tggccatgta atcgagaata tcaccgaagt tacgcctgtt 660

cctgcacacc aattacagct cagcatcgat gagcgcctgc agacggtcac tgaagatgcg 720

ttggacaatg ccgttacgtg gaataaagcg gaatcaggag ccgcagtgct ggtcaacatt 780

gctaccggtg aaatactgtc catggcgagc tttcctgact tcaaccccaa taaccgtgac 840

ggcgcggtac tggatgattt tcgtaaccgt gcgataagcg atacgtttga acccggttca 900

accgttaaac cgctggtgat catgactgcg ctccagcagg ggatagtaca accggacagc 960

gtgattgata cccatccttt ttaccttgat ggacatcgca ttcgcgacgt cggattttac 1020

ccggagctgt cgttgaccgg aattttgcag aaatcgagcg ataccggcgt gtcgcacctt 1080

tcactggcta tgcctgttca aaagttgctg gatacctata agagctttgg ttttggcgat 1140

ccaaccggac tgggattaac cggtgaaagt agcggtttaa tgcctcaacg ccgctactgg 1200

agcgatttgg atcgcgcgac ctttgcattc ggctatggat taatggtaac gccgctgcag 1260

ttggcgcacg tatatgcaac cattggtagt tttggtatct atcgacccct ttcgattacc 1320

cgtattgacc cgccggtaat cggtaccaga gtgatgccag aggcgctggt tcaccaggtt 1380

gaacatatga tggaaagcgt cgcgttaccc ggtggtgggg gaactaaagc cgcggtcaga 1440

gattatcgcg tagcggttaa aacaggaacg gcaaagaaaa ttggtgatga cggtaagtat 1500

gtcgataaat acgtggccta cacggcaggc gttgcgccag cgagcgaccc gaaatttgca 1560

ctggtggtcg tcatcaataa tccgcaaaac ggtgcttatt atggtggggc tgtctctgcg 1620

cctgtcttca gccagataat gggtgatgtc ctgcgtctgg agaacgttaa accggatggt 1680

atgcctgccg actctgatca tctgctggtg atgcacggca gcaacgtatc ctcgccgtcg 1740

ctgtaa 1746

<210> 10
<211> 581
<212> PRT
<213> Citrobacter freundii

<400> 10

Met Lys Lys Lys Asn Asp Gly Asp Ala Arg Asn Phe Thr Pro Val Arg
1               5                   10                  15

Phe Ala Leu Leu Cys Val Ala Ile Leu Leu Ser Leu Gly Leu Leu Leu
            20                  25                  30

Gly Arg Val Ala Trp Leu Gln Ile Val Thr Pro Thr Ser Leu Val Lys
        35                  40                  45

Gln Glu Asp Met Arg Ser Leu Arg Glu Val Thr Ile Ala Ser Pro Arg
    50                  55                  60

Gly Met Ile Thr Asp Arg Glu Gly Arg Pro Leu Ala Val Ser Val Pro
65                  70                  75                  80

Val Asn Ala Val Trp Ala Asp Pro Lys Thr Ile Ile Ser Lys Gly Gly
                85                  90                  95

Val Gly Tyr Asn Glu Arg Trp Gln Ala Leu Ala Arg Ala Leu His Leu
            100                 105                 110

Ser Leu Ser Thr Leu Ala Glu Arg Val Asn Ser Asn Pro Gly Gly Arg
        115                 120                 125

Phe Ile Tyr Leu Ala Arg Gln Ile Ser Pro Gln Gln Ala Glu Trp Val
    130                 135                 140

Asp Asp Leu Asn Leu Pro Gly Ile Asn Leu Arg Glu Glu Ser Arg Arg
145                 150                 155                 160

Phe Tyr Pro Ala Gly His Val Ala Ala Asn Leu Ile Gly Phe Thr Asn
                165                 170                 175

Ile Asp Gly Gln Gly Ile Glu Gly Leu Glu Lys Ser Phe Asn Asn Gln
            180                 185                 190

Leu Met Gly Lys Pro Gly Ser Arg Leu Val Arg Lys Asp Lys Phe Gly
        195                 200                 205

His Val Ile Glu Asn Ile Thr Glu Val Thr Pro Val Pro Ala His Gln
    210                 215                 220

Leu Gln Leu Ser Ile Asp Glu Arg Leu Gln Thr Val Thr Glu Asp Ala
225                 230                 235                 240

Leu Asp Asn Ala Val Thr Trp Asn Lys Ala Glu Ser Gly Ala Ala Val
                245                 250                 255

Leu Val Asn Ile Ala Thr Gly Glu Ile Leu Ser Met Ala Ser Phe Pro
            260                 265                 270

Asp Phe Asn Pro Asn Asn Arg Asp Gly Ala Val Leu Asp Asp Phe Arg
        275                 280                 285

Asn Arg Ala Ile Ser Asp Thr Phe Glu Pro Gly Ser Thr Val Lys Pro
    290                 295                 300

Leu Val Ile Met Thr Ala Leu Gln Gln Gly Ile Val Gln Pro Asp Ser
305                 310                 315                 320

Val Ile Asp Thr His Pro Phe Tyr Leu Asp Gly His Arg Ile Arg Asp
                325                 330                 335

Val Gly Phe Tyr Pro Glu Leu Ser Leu Thr Gly Ile Leu Gln Lys Ser
            340                 345                 350

Ser Asp Thr Gly Val Ser His Leu Ser Leu Ala Met Pro Val Gln Lys
        355                 360                 365

Leu Leu Asp Thr Tyr Lys Ser Phe Gly Phe Gly Asp Pro Thr Gly Leu
    370                 375                 380

Gly Leu Thr Gly Glu Ser Ser Gly Leu Met Pro Gln Arg Arg Tyr Trp
385                 390                 395                 400

Ser Asp Leu Asp Arg Ala Thr Phe Ala Phe Gly Tyr Gly Leu Met Val
                405                 410                 415

Thr Pro Leu Gln Leu Ala His Val Tyr Ala Thr Ile Gly Ser Phe Gly
            420                 425                 430

Ile Tyr Arg Pro Leu Ser Ile Thr Arg Ile Asp Pro Pro Val Ile Gly
        435                 440                 445

Thr Arg Val Met Pro Glu Ala Leu Val His Gln Val Glu His Met Met
    450                 455                 460

Glu Ser Val Ala Leu Pro Gly Gly Gly Gly Thr Lys Ala Ala Val Arg
465                 470                 475                 480

Asp Tyr Arg Val Ala Val Lys Thr Gly Thr Ala Lys Lys Ile Gly Asp
                485                 490                 495

Asp Gly Lys Tyr Val Asp Lys Tyr Val Ala Tyr Thr Ala Gly Val Ala
            500                 505                 510

Pro Ala Ser Asp Pro Lys Phe Ala Leu Val Val Val Ile Asn Asn Pro
        515                 520                 525

Gln Asn Gly Ala Tyr Tyr Gly Gly Ala Val Ser Ala Pro Val Phe Ser
    530                 535                 540

Gln Ile Met Gly Asp Val Leu Arg Leu Glu Asn Val Lys Pro Asp Gly
545                 550                 555                 560

Met Pro Ala Asp Ser Asp His Leu Leu Val Met His Gly Ser Asn Val
                565                 570                 575

Ser Ser Pro Ser Leu
            580

<210>  11
<211>  70
<212>  DNA
<213>  Artificial Sequence

<220>
<223>  KO STPBP2 FW primer

<400>  11
caaccaccgg gttttccgca ggcaaatggg tgttgttgtc gcccagcatg gtgtaggctg        60

gagctgcttc                                                               70

<210>  12
<211>  70
<212>  DNA
<213>  Artificial Sequence

<220>
<223>  KO STPBP2 RV primer

<400>  12
atgaaacgac aaaattcttt tcgtgactat acggctgagt ccgcactgtt attccgggga        60

tccgtcgacc                                                               70

<210>  13
<211>  25
<212>  DNA
<213>  Artificial Sequence

<220>
<223>  pbp2 flanking FW primer

<400>  13
ggatcgatat gaattttatc ccaga                                              25

<210>  14
<211>  25
<212>  DNA
<213>  Artificial Sequence

<220>
<223>  pbp2 flanking RV primer

<400>  14
cgggcgtgga gcatcacaac caacc                                          25


<210>  15
<211>  40
<212>  DNA
<213>  Artificial Sequence

<220>
<223>  PBP2SAL S326A FW primer

<400>  15
aagggcttta tcctccggcg gcaacggtta aaccctatat                          40


<210>  16
<211>  40
<212>  DNA
<213>  Artificial Sequence

<220>
<223>  PBP2SAL S326A RV primer

<400>  16
atatagggtt taaccgttgc cgccggagga taaagccctt                          40


<210>  17
<211>  70
<212>  DNA
<213>  Artificial Sequence

<220>
<223>  Exchange PBP3SAL by PBP3 1 primer

<400>  17
ccgtttcccg ttaaatcaat cacctgaaaa atgattcggc tggagatcag atgaaagcag    60

cggcaaaaac                                                           70


<210>  18
<211>  40
<212>  DNA
<213>  Artificial Sequence

<220>
<223>  Exchange PBP3SAL by PBP3 2 primer

<400>  18
gaagcagctc cagcctacac ttacgatctg ccacctgttc                          40


<210>  19
<211>  40
<212>  DNA
<213>  Artificial Sequence

<220>

<223>  Exchange PBP3SAL by PBP3 3 primer

<400>  19
gaacaggtgg cagatcgtaa gtgtaggctg gagctgcttc                              40


<210>  20
<211>  70
<212>  DNA
<213>  Artificial Sequence

<220>
<223>  Exchange PBP3SAL by PBP3 4 primer

<400>  20
gggcgctaaa gggcgcaagt gtaacgcgaa ttgcgccccg ggaaaatcct attccgggga        60

tccgtcgacc                                                               70


<210>  21
<211>  75
<212>  DNA
<213>  Artificial Sequence

<220>
<223>  Exchange PBP2SAL by PBP2 1 primer

<400>  21
ggcgctgacc gccccgtatc attaggcggc ggattgttca ggtacaggta atgaaacgac        60

aaaattcttt tcgtg                                                         75


<210>  22
<211>  45
<212>  DNA
<213>  Artificial Sequence

<220>
<223>  Exchange PBP2SAL by PBP2 2 primer

<400>  22
gaagcagctc cagcctacac ttattggtcc tccgccgctg caacc                        45


<210>  23
<211>  45
<212>  DNA
<213>  Artificial Sequence

<220>
<223>  Exchange PBP2SAL by PBP2 3 primer

<400>  23
ggttgcagcg gcggaggacc aataagtgta ggctggagct gcttc                        45


<210>  24
<211>  70
<212>  DNA
<213>  Artificial Sequence

<220>
<223>  Exchange PBP2SAL by PBP2 4 primer

<400>  24
atgcagatat tccggccgta tccttgtctg atggcgcttt gcttatttga attccgggga      60

tccgtcgacc                                                              70


<210>  25
<211>  35
<212>  DNA
<213>  Artificial Sequence

<220>
<223>  fwSpeI-PBP2 primer

<400>  25
cgctactagt atgaaacgac aaaattcttt tcgtg                                  35


<210>  26
<211>  35
<212>  DNA
<213>  Artificial Sequence

<220>
<223>  revSpe-PBP2 primer

<400>  26
ccctactagt ttattggtcc tccgccgctg caacc                                  35


<210>  27
<211>  35
<212>  DNA
<213>  Artificial Sequence

<220>
<223>  fwSpeI-PBP2* primer

<400>  27
cgctactagt atgactttta aagactttga tgcgg                                  35


<210>  28
<211>  35
<212>  DNA
<213>  Artificial Sequence

<220>
<223>  revSpeI-PBP2*  primer

<400>  28
ccctactagt ctatggcgcc tgatccggct gtgtg                                  35


<210>  29
<211>  25
<212>  DNA
<213>  Artificial Sequence

<220>

<223>  pAC FW primer

<400>  29
cggccctcat tcgtgcgctc tagga                                          25


<210>  30
<211>  22
<212>  DNA
<213>  Artificial Sequence

<220>
<223>  pAC-sec primer

<400>  30
cataatgggg aaggccatcc ag                                             22


<210>  31
<211>  25
<212>  DNA
<213>  Artificial Sequence

<220>
<223>  pFUS FW primer

<400>  31
gcacggcgtc acactttgct atgcc                                          25


<210>  32
<211>  25
<212>  DNA
<213>  Artificial Sequence

<220>
<223>  pFUS REV primer

<400>  32
gctaagatct cccatatgta tatct                                          25


<210>  33
<211>  60
<212>  DNA
<213>  Artificial Sequence

<220>
<223>  FLAG-2*-Fw primer

<400>  33
tgatccacag gctgatacca cacagccgga tcaggcgcca gactacaaag accatgacgg    60


<210>  34
<211>  60
<212>  DNA
<213>  Artificial Sequence

<220>
<223>  FLAG-2*-Rv primer

<400>  34

tccggccgta tccttgtctg atggcgcttt gcttatttga catatgaata tcctccttag     60

<210>  35
<211>  35
<212>  DNA
<213>  Artificial Sequence

<220>
<223>  fwSpeI-PBP3Citro primer

<400>  35
cgctactagt gtgaaaaaga aaaacgacgg cgacg                                35

<210>  36
<211>  35
<212>  DNA
<213>  Artificial Sequence

<220>
<223>  revPvuI-PBP3Citro primer

<400>  36
ccctcgatcg ttacagcgac ggcgaggata cgttg                                35

<210>  37
<211>  22
<212>  DNA
<213>  Artificial Sequence

<220>
<223>  FL-2* Fw primer

<400>  37
aggcctgact ccggataacg ga                                              22

<210>  38
<211>  21
<212>  DNA
<213>  Artificial Sequence

<220>
<223>  FL-2* Rv primer

<400>  38
acctctatgc cttacgggca g                                               21

<210>  39
<211>  70
<212>  DNA
<213>  Artificial Sequence

<220>
<223>  KO PBP2* Fw primer

<400>  39
ggcgctgacc gccccgtatc attaggcggc ggattgttca ggtacaggta gtgtaggctg     60

gagctgcttc                                                            70

<210>  40
<211>  70
<212>  DNA
<213>  Artificial Sequence

<220>
<223>  KO PBP2* Rv primer

<400>  40
atgcagatat tccggccgta tccttgtctg atggcgcttt gcttatttga attccgggga        60

tccgtcgacc                                                                70


<210>  41
<211>  22
<212>  DNA
<213>  Artificial Sequence

<220>
<223>  FL-pbp2*-FW primer

<400>  41
aggcctgact ccggataacg ga                                                  22


<210>  42
<211>  21
<212>  DNA
<213>  Artificial Sequence

<220>
<223>  FL-pbp2*-RV primer

<400>  42
acctctatgc cttacgggca g                                                   21

## Description

The invention relates to an *in vitro* screening method to identify inhibitors that block bacterial growth inside eukaryotic cells ("intracellular growth") and uses thereof as medicament, preferably as antibiotics for the treatment and/or prevention of intracellular bacterial infections.

### BACKGROUND ART

Antibiotic resistance is one of the major problems in human and animal medicine. Although in many cases the origin of the bacterial resistance is clear, some pathogens remain difficult to eradicate, pointing to bacterial mechanisms of resistance that remain unknown. This phenomenon has been repeatedly reported in infections caused by intracellular bacterial pathogens.

Bacterial infections by antibiotic-resistant bacteria are a major contributor to global morbidity and mortality, and due to the wide-spread use of antibiotics and the lack of new antibiotics, the numbers of highly resistant bacteria continue to increase across the globe. Amongst such are the intracellular infections caused by *Salmonellae,* including those caused by the highly invasive *S*. *enterica serovar* Typhi and invasive non-typhoidal serovars, including serovars Typhiumurium and Enteritidis. Bloodstream invasive infections caused by *Salmonellae* are also highly frequent in children, leading to considerable mortality particularly in underdeveloped areas. Invasive (systemic) salmonellosis are normally treated with antibiotics such as aminopenicillins, cotrimoxazole, fluoroquinolones and, increasingly, third-generation cephalosporins or monobactams because of frequent resistance to the other agents. Aminopenicillins, third generation cephalosporins and monobactams are all beta-lactam antibiotics that have penicillin binding proteins (PBPs) as targets. PBPs are enzymes involved in the last biosynthetic steps of the peptidoglycan (PG), main component of the bacterial cell wall. PBPs cleave the terminal D-Ala-D-Ala bond present in stem peptide of the PG to incorporate precursor units into the nascent PG via a D-D-transpeptidation reaction. Beta-lactams bind covalently to PBPs leading to an irreversible enzyme blockage. In the model Gram-negative bacterium *Escherichia coli,* PBP2 and PBP3 are essential enzymes involved in PG assembly during cell elongation and division, respectively.

*Salmonellae* evolved to adapt to the intracellular environment of eukaryotic cells and this explains why these organisms are frequently invasive. Bacteria invade enterocytes and dendritic cells and later by macrophages in the submucosa. The intracellular character of the infections caused by these pathogens also accounts for frequent persistent bacteraemia, chronic carriage, and relapses mostly in children despite the widespread use of beta-lactams. Interestingly, *Salmonellae* are sensitive to beta lactam antibiotics *in vitro* but display reduced sensitivity *in vivo,* being this an important medical problem. This therapeutic failure has been associated to reduced accumulation of the beta-lactam, particularly penicillin, into the acidic compartments of eukaryotic cells colonized by the pathogen. Lack of beta-lactams accumulation may occur due to their weak acid character. In addition, the establishment by the pathogen of a persistent dormant-like state in intracellular locations limits beta-lactam bacteriolytic action.

Based on these observations, there is a need in the art to identify proteins that the pathogen uses specifically in the intracellular environment and responsible for antibiotic resistance. These proteins should be suitable for use in drug screening assays and, as consequence, targeted for the treatment of intracellular bacterial infections.

### SUMMARY OF THE INVENTION

The present invention discloses that pathogenic bacteria, preferably Gram-negative bacteria, more preferably *Salmonella spp,* and more preferably *Salmonella* species *enterica* subspecies I serovar Typhimurium (*S*. Typhimurium) becomes innately resistant to beta-lactams when it naturally adapts to the hostile acidic intra-phagosomal environment inside eukaryotic cells. This character is not induced by drug exposure and is acquired due to the substitution, in intracellular bacteria, of the main beta-lactam targets, the PBP2 (SEQ ID NO: 6) and PBP3 (SEQ ID NO: 8), by two new pathogen-specific enzymes that have low affinity for these antibiotics, PBP2SAL (SEQ ID NO: 2) and PBP3SAL (SEQ ID NO: 4), respectively. Thus, the intracellular eukaryotic cell niche promotes in the pathogen an INtracellularly-Driven Antibiotic Resistance (INDAR) phenotype undetectable in standard *in vitro* culture media. This unprecedent and surprising mechanism, in which traditional targets, such as PBP2 (SEQ ID NO: 6) and/or PBP3 (SEQ ID NO: 8) are substituted by PBP2SAL (SEQ ID NO: 2) and/or PBP3SAL (SEQ ID NO: 4), respectively without selective pressure as part of a natural adaptive process to a specific niche, is a foundation for therapies aimed to eradicate persistent intracellular infections.

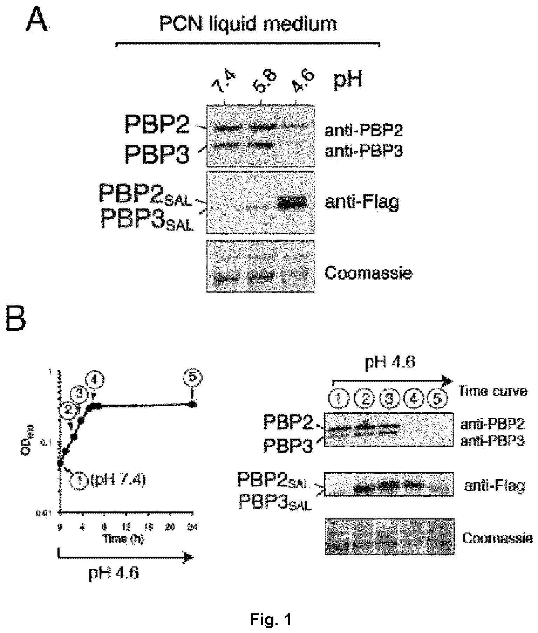

In the present invention it is showed that acid pH and nutrient limitation are signals perceived by intracellular pathogenic bacteria, preferably by *S*. Typhimurium to switch PBP2 and PBP3 expression by PBP2SAL and PBP3SAL expression **(****Fig. 1****)**. This change may play an instrumental role in its adaptation to the phagosomal niche. In addition, such switch results in 'unexpected' resistance to beta-lactams inhibiting PBP2 and PBP3 without any selective pressure imposed by exposure to the antibiotic(s). This unprecedent mechanism of antibiotic resistance, which it is named in the present invention as INDAR, is therefore transitory as it is acquired specifically within the infected eukaryotic host cell. The linkage between the INDAR phenotype and acidity is extreme in the case of PBP3SAL, an enzyme unable to bind beta-lactams at neutral pH. The appearance of PBP2SAL and PBP3SAL in intracellular bacteria, more preferably in intracellular *Salmonellae* has therefore important consequences for antibiotic effectiveness, rendering these pathogens much less susceptible when they colonize the phagosome.

Thus, the inventors show that, as part of its adaptive program to the intracellular lifestyle, pathogenic bacteria, i.e. *Salmonellae* produces two PBPs *de novo,* PBP2SAL (SEQ ID NO: 2) and PBP3SAL (SEQ ID NO: 4) that substitute pre-existing enzymes PBP2 (SEQ ID NO: 6) and PBP3 (SEQ ID NO:8), respectively. Moreover, the inventors show that the poor effectiveness of beta-lactams to eradicate *Salmonellae* in the intracellular niche is linked to this replacement of PBP2/PBP3 (natural targets) by PBP2SAL/PBP3SAL, respectively that takes place within the infected host cell. The PBPs responding to intracellular cues, PBP2SAL and PBP3SAL, exhibit low affinity for beta-lactam antibiotics. These features render intracellular *Salmonellae* innately resistant to beta-lactams by an unprecedented mechanism that is not stimulated or selected upon drug exposure, which we refer in the present invention as INDAR.

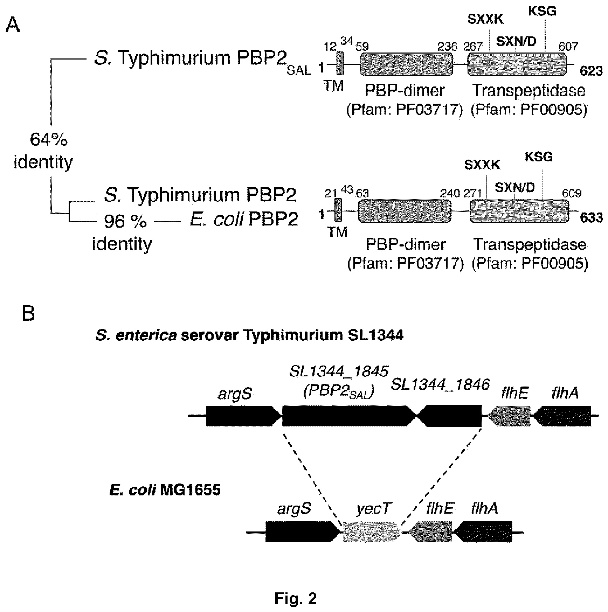

In a preferred embodiment of the present invention, the PBP2SAL and PBP3SAL proteins of S. Typhimurium have 64% and 63% amino acid identity to PBP2 and PBP3 respectively of S. Typhimurium, involved in cell elongation and division, respectively. Consistent with a role in peptidoglycan metabolism, PBP2SAL and PBP3SAL bear a transpeptidase functional domain (Pfam:PF00905) and conserve the residues critical for catalysis **(****Fig. 2****).**

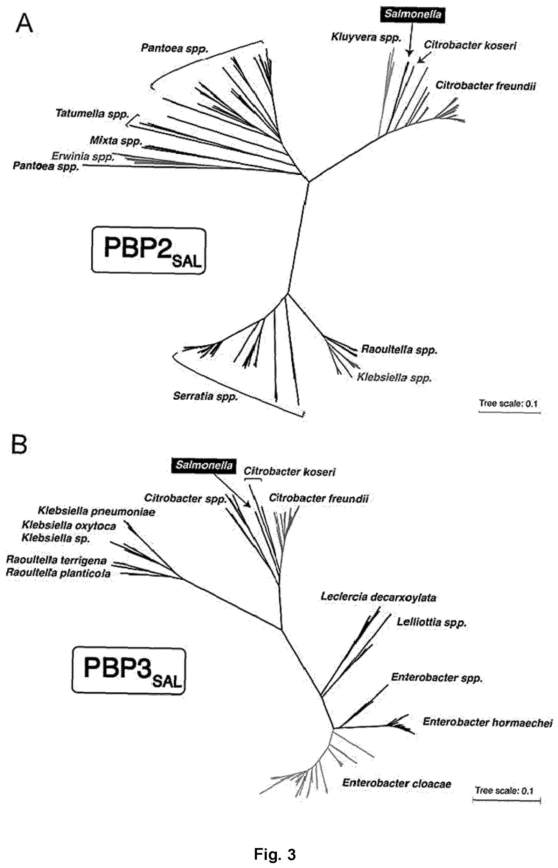

Importantly, PBP2 and PBP3 inhibition by beta-lactams results in shape alterations and cell death in Gram-negative bacteria. BLAST analyses reveal that PBP2SAL and PBP3SAL are conserved in all *Salmonellae* and that protein homologs with ≥80% identity and ≥95% protein coverage are also present in opportunistic bacterial pathogens of environmental origin **(****Fig. 3****).**

In S. Typhimurium, PBP2SAL and PBP3SAL expression responds to a phagosomal environmental cue like acid pH and promotes pathogen division inside eukaryotic cells. In this sense, all type of invasive salmonellosis (typhoidal and non-typhoidal) progress with bacteria colonizing an intracellular niche. New antibiotics inhibiting PBP2SAL and PBP3SAL will specifically target the intra-phagosomal sanctuary, reducing the risk of invasion and avoiding relapses in salmonellosis and infections caused by this pathogen or opportunistic pathogens harbouring PBP2SAL and PBP3SAL homologs.

In summary, the results of the invention allow new therapeutic approaches to intracellular bacterial infections to be developed, through the use of pharmaceutical compositions comprising active principles that inhibit the expression and/or activity of genes and/or their encoded proteins responsible for the INDAR phenotype.

In view of the foregoing, the present invention allows the skilled person in the art to identify those compounds which are useful as medicine, preferably as antibiotic because inhibit PBP2SAL and/or PBP3SAL. These new compounds will specifically target the intra-phagosomal sanctuary, reducing the risk of invasion and avoiding relapses in systemic infections caused by *Salmonellae* and other Gram-negative bacterial pathogens.

Therefore, one aspect of the invention relates to an INDAR inhibitor, hereinafter "inhibitor of the invention", for use as medicament.

The terms "INDAR" or "INtracellularly-Driven Antibiotic Resistance", used interchangeably in the present invention refer to a set of proteins encoded by the INDAR genes.

The term "INDAR gene" used in the present invention refers to a gene that is activated by pathogenic bacteria, preferably Gram-negative, inside eukaryotic cells and encodes a protein with lower affinity for antibiotic currently in use, preferably for beta-lactam antibiotics, therefore promoting resistance. This term comprises the gene named as "PBP2SAL" and appearing in the databases also named as *mrdA, pbpA, STM1910, SL1344_1845, MRDA_SALTY, PBP2_SALTY*. In the context of the present invention, *PBP2SAL* is also defined by a nucleotide or polynucleotide sequence, which constitutes the coding sequence of the PBP2SAL protein, and which would comprise various variants from:
a) nucleic acid molecules encoding a polypeptide comprising the amino acid sequence of SEQ ID NO: 2,
b) nucleic acid molecules whose chain complement it hybrid with the polynucleotide sequence of a),
c) nucleic acid molecules whose sequence differs from a) and/or b) due to the degeneracy of the genetic code,
nucleic acid molecules encoding a polypeptide comprising the amino acid sequence with an identity of at least 80%, 85%, 90%, 91%, 92%, 93%, 94%, 95%, 96%, 97%, 98% or 99% with SEQ ID NO: 2. in that the polypeptide encoded by said nucleic acids possesses the activity and structural characteristics of the PBP2SAL protein. Among these nucleic acid molecules is the SEQ ID NO: 1.

Furthermore, the term INDAR gene also comprises the gene *"PBP3SAL"* also called in the literature as *ftsI2, ftsl, STM1836* or *SL1344_1765.* In the context of the present invention, *PBP3SAL* is also defined by a nucleotide or polynucleotide sequence, which constitutes the coding sequence of the PBP3SAL protein, and which would comprise various variants from:
a) nucleic acid molecules encoding a polypeptide comprising the amino acid sequence of SEQ ID NO: 4,
b) nucleic acid molecules whose chain complement it hybrid with the polynucleotide sequence of a),
c) nucleic acid molecules whose sequence differs from a) and/or b) due to the degeneracy of the genetic code,
d) nucleic acid molecules encoding a polypeptide comprising the amino acid sequence with an identity of at least 80%, 85%, 90%, 91%, 92%, 93%, 94%, 95%, 96%, 97%, 98% or 99% with SEQ ID NO: 4. in that the polypeptide encoded by said nucleic acids possesses the activity and structural characteristics of the PBP3SAL protein. Among these nucleic acid molecules is the SEQ ID NO: 3.

The present invention also refers to the homologs, orthologs, paralogs of the genes and proteins encoding thereof, selected form the list: PBP2SAL, PBP3SAL, PBP2 and PBP3, and fragments and variations thereof.

As used herein, the term "homologous" or "homolog" is known in the art and refers to related sequences that share a common ancestor or family member and are determined based on the degree of sequence identity. We define homolog as protein having at least a minimum of 80%, preferably 85%, 90%, 91%, 92%, 93%, 94%, 95%, 96%, 97%, 98% or 99% identity in a stretch that covers at least 95% or more of the protein length. The terms "homology", "homologous", "substantially similar" and "corresponding substantially" are used interchangeably herein. Homologs usually control, mediate, or influence the same or similar biochemical pathways, yet particular homologs may give rise to differing phenotypes. It is therefore understood, as those skilled in the art will appreciate, that the invention encompasses more than the specific exemplary sequences. These terms describe the relationship between a gene found in one species or subspecies and the corresponding or equivalent gene in another species or subspecies. For purposes of this invention homologous sequences are compared.

The term "homolog" is sometimes used to apply to the relationship between genes separated by the event of speciation (see "ortholog") or to the relationship between genes separated by the event of genetic duplication (see "paralog").

The term "ortholog" refers to genes in different species that evolved from a common ancestral gene by speciation. Normally, orthologs retain the same function in the course of evolution. Identification of orthologs is critical for reliable prediction of gene function in newly sequenced genomes.

The term "paralog" refers to genes related by duplication within a genome or gene copies that were acquired by horizontal gene transfer encoding similar proteins to that encoded in the receptor genome. While orthologs generally retain the same function in the course of evolution, paralogs can evolve new functions, even if these are related to the original one.

"Homologous sequences" or "homologs" or "orthologs" are thought, believed, or known to be functionally related. A functional relationship may be indicated in any one of a number of ways, including, but not limited to: (a) degree of sequence identity and/or (b) the same or similar biological function. Preferably, both (a) and (b) are indicated. The degree of sequence identity may vary, but in one embodiment, is at least 50% (when using standard sequence alignment programs known in the art), at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 98.5%, or at least 99%, or at least 99.5%, or at least 99.8%, or at least 99.9%. Homology can be determined using software programs readily available in the art, such as those discussed in Current Protocols in Molecular Biology (F. M. Ausubel et al., eds., 1987) Supplement 30, section 7.718, Table 7.71. Some alignment programs are MacVector (Oxford Molecular Ltd, Oxford, U.K.) and ALIGN Plus (Scientific and Educational Software, Pennsylvania). Other non-limiting alignment programs include Sequencher (Gene Codes, Ann Arbor, Mich.), AlignX, and Vector NTI (Invitrogen, Carlsbad, Calif).

In a preferred embodiment, an orthologous of *PBP3SAL* gene from *Citrobacter* spp, more preferably from *Citrobacter freundii* is defined by a nucleotide or polynucleotide sequence, which constitutes the coding sequence of the PBP3SAL protein from this organism, and which would comprise various variants from:
a) nucleic acid molecules encoding a polypeptide comprising the amino acid sequence of SEQ ID NO: 10,
b) nucleic acid molecules whose chain complement it hybrid with the polynucleotide sequence of a),
c) nucleic acid molecules whose sequence differs from a) and/or b) due to the degeneracy of the genetic code,
d) nucleic acid molecules encoding a polypeptide comprising the amino acid sequence with an identity of at least 80%, 85%, 90%, 91%, 92%, 93%, 94%, 95%, 96%, 97%, 98% or 99% with SEQ ID NO: 10. in that the polypeptide encoded by said nucleic acids possesses the activity and structural characteristics of the PBP3SAL protein. Among these nucleic acid molecules is the SEQ ID NO: 9.

In the art, the terms "identity" and "similarity" mean the degree of polypeptide or polynucleotide sequence relatedness which are determined by matching a query sequence and other sequences of preferably the same type (nucleic acid or protein sequence) with each other. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein might be evolutionarily neutral mutations which do not affect its global structure or its functionality

As used herein the term "wild-type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

In another particular embodiment, the INDAR gene *PBP2SAL* comprises a nucleotide sequence having a sequence identity of, at least, 100% with the sequence SEQ ID NO: 1. In a most particular embodiment, the INDAR gene PBP2SAL consists of the nucleotide sequence SEQ ID NO: 1. In another particular embodiment, the INDAR protein PBP2SAL comprises an amino acid sequence having a sequence identity of, at least, 100% with the sequence SEQ ID NO: 2. In a most particular embodiment, the INDAR protein PBP2SAL consists of the amino acid sequence SEQ ID NO: 2.

In another particular embodiment, the INDAR gene *PBP3SAL* comprises a nucleotide sequence having a sequence identity of, at least, 100% with the sequence SEQ ID NO: 3. In a most particular embodiment, the INDAR gene PBP3SAL consists of the nucleotide sequence SEQ ID NO: 3. In another particular embodiment, the INDAR protein PBP3SAL comprises an amino acid sequence having a sequence identity of, at least, 100% with the sequence SEQ ID NO: 4. In a most particular embodiment, the INDAR protein PBP3SAL consists of the amino acid sequence SEQ ID NO: 4.

The present invention comprises the use of an INDAR inhibitor as medicament. The term "INDAR inhibitor" refers to any molecule capable of completely or partially inhibiting the INDAR phenotype, either by preventing the expression product of said gene from being produced (interrupting the INDAR gene transcription and/or blocking the translation of the mRNA coming from the INDAR gene expression) or by directly inhibiting the INDAR protein activity. Methods for decreasing/abrogating the expression of the gene encoding the INDAR protein include, without being limited to, editing technologies such as CRISPR/cas9 or Cas9 nickase technology. By way of non-limiting illustration, INDAR inhibitors suitable for use in the present invention include chemical compounds, antisense oligonucleotides, interference RNAs (siRNAs), catalytic RNAs or specific ribozymes and inhibitory antibodies. Thus, in a particular embodiment, the INDAR inhibitor is selected from the group consisting of a chemical compound that binds the INDAR protein, an INDAR specific siRNA, an INDAR specific antisense oligonucleotide, an INDAR specific ribozyme, and an inhibitory INDAR specific antibody.

The term "medicament", as it is used in the present description, relates to any substance or combination of substances having properties for the treatment or prevention of diseases in organisms, preferably human beings, or that may be used or administered to the organisms, preferably, mammals and/or avian, more preferably human beings, with the aim of restoring, correcting or modifying the physiological functions by exercising a pharmacological, immunological or metabolic action. The medicament of the invention may be used both alone and in combination with other medicaments or compositions to improve cognitive performance and/or promote cognitive development by way of combined therapy, and can be administered simultaneously or at different time intervals.

In a preferred embodiment, the INDAR inhibitor is an antibiotic compound.

The term "antibiotic", as it is used in the present description, relates to a substance or compound often produced by or derived from certain fungi, bacteria, and other organisms, or additionally, synthetically produced, that can destroy or inhibit the growth of pathogenic microorganisms, preferably pathogenic bacteria.

The term pathogenic bacteria comprise defined bacterial groups within Gram-negative and Gram-positive bacteria. Gram-negative bacteria are a group of bacteria having a cell wall composed of a thin layer of peptidoglycan. Gram-negative bacteria lose the crystal violet stain and take the colour of the red counterstain in Gram's Method of staining. Gram-negative bacteria include most of the bacteria normally causing enteric infections. Exemplary Gram-negative bacteria include *Escherichia spp., Salmonella spp., Citrobacter spp., Klebsiella spp., Enterobacter spp., Lelliottia spp., Cedecea spp., Raoultella spp., Erwinia spp., Leclercia spp., Kluyvera spp., Mixta* spp., *Pantoea spp, Pluribacter spp., Rosenbergiella spp., Morganella spp., Tatumella spp., Serratia spp., Acinetobacter spp.,* and *Haemophilus spp.*

In a more preferred embodiment, the *Salmonella genus comprises the species selected from: S. enterica and S. bongori.*

In a more preferred embodiment, the *S*. *enterica* species comprises the subspecies selected from the list consisting of: *S*. *enterica subspecies I (enterica), II (salamae), IIIa (diarizonae), IIIb (arizonae), IV (houtenae), and VI (indica).* In a more preferred embodiment, the *S*. *enterica* subspecies *enterica* (I) comprises more than 1,500 serovars within this subspecies, including serovar Typhimurium, Typhi, and Enteritidis.

In a more preferred embodiment, the *Citrobacter spp* comprises the species selected from the list consisting of: *C. koseri, C. freundii, C. youngae, C. braakii, C. werkmanii, C. murliniae, C. gillenii, C. sedlakii, C. pasteurii, C. eropaeus, C. amalonaticus, C. farmeri, C. rodentium* and *C. portucalensis.*

In a more preferred embodiment, the *Enterobacter spp.* comprises the species selected from the list consisting of: *E. cloacae, E. hormaechei, E. roggenkampii, E. chengduensis, E. kobei, E. asburiae, E. sichuanensis, E. ludwigii, E. huaxiensis, E. soli, E. mori, E. tabaci, E. bugandensis* and *E. lignolyticus.*

In a more preferred embodiment, the *Klebsiella spp.* comprises the species selected from: *K. pneumoniae, K. oxytoca, K. aerogenes, K. michiganensis*, *K. variicola, K. pseudovariicola* and *K. quasiopneumoniae.*

In a more preferred embodiment, the *Lelliottia spp.* comprises the species selected from the list consisting of: *L. nimipressuralis, L. amnigena, L. jeotgali* and *L. aquatilis.*

In a more preferred embodiment, the *Cedecea spp.* comprises the species *C. lapagei.*

In a more preferred embodiment, the *Leclercia spp.* comprises the species *L. adecarboxylata.*

In a more preferred embodiment, the *Raoultella spp.* comprises the species selected from the list consisting of: *R. ornithinolytica, R. terrigena* and *R. planticola.*

In a more preferred embodiment, the *Kluyvera spp.* comprises the species selected from the list consisting of: *K. intermedia, K. georgiana, K. ascorbate* and *K*. *cyrocrescens.*

In a more preferred embodiment, the *Pantoea spp.* comprises the species selected from the list consisting of: *P. septica, P. vagans, P. latae, P. cypripedii, P. dispersa, P. stewartii, P. ananatis, P. rodasii, P. allii, P. wallisii, P. deleyi, P. brenneri, P. agglomerans, P. eucalypti, P. eucrina, P. antophila, P. endophytica* and *P*. *rwandensis.*

In a more preferred embodiment, the *Tatumella spp.* comprises the species selected from the list consisting of: *T. morbirosei, T. saanichensis* and *T. ptyseos.*

In a more preferred embodiment, the *Erwinia spp.* comprises the species selected from the list consisting of: *E. typographi, E. billingiae, E. gerundensis, E. tracheiphila* and *E. mallotivora.*

In a more preferred embodiment, the *Mixta spp.* comprises the species selected from the list consisting of: *M. calida, M. gaviniae* and *M*. *theicola.*

In a more preferred embodiment, the *Serratia spp.* comprises the species selected from the list consisting of: *S*. *marcenses, S. fonticola, S. nematodiphila, S. plymuthica, S. proteamaculans, S. liquefaciens, S. odorifera, S. grimesii, S. ureilytica* and *S. rubidaea.*

The term "treatment", as it is used in the present invention, relates to combating the effects caused as a consequence of the disease or pathological condition of interest in a subject (preferably mammals and/or avian and, more preferably, a human) including:
(i). inhibit the disease or pathological condition, i.e. stop the development thereof;
(ii). alleviate the disease or pathological condition, i.e. cause the regression of the disease or pathological condition or its symptomatology;
(iii). stabilize the disease or pathological condition.

In the present invention, the condition or disorder to be treated are intracellular bacterial infections. Specifically, bacterial infections which become innately resistant to antibiotics, preferably beta-lactams when it naturally adapts to the hostile intraphagosomal environment inside eukaryotic cells. In a more preferred embodiment, the condition treated are systemic infections caused by bacteria harbouring the INDAR genes. In a more preferred embodiment, the conditions treated are selected from the list consisting of: salmonellosis, typhoid fever, non-typhoid fever, gastroenteritis, arthritis, polymicrobial infections, respiratory infections, systemic infections and asymptomatic intracellular persistent and chronic infections.

The term "prevention" (or "prevent"), as it is used in the present invention, consists of avoiding the onset of the disease, i.e. avoiding the disease or pathological condition in a subject (preferably mammals and/or avian and, more preferably, a human), in particular, when said subject has a predisposition to the pathological condition but has not been diagnosed yet. In a preferred embodiment, the term prevention refers to the capacity of the INDAR inhibitor to minimize or hinder the progression of a disease, condition or disorder in a subject. In the present invention, the condition or disorder to be prevented are intracellular bacterial infections progressing with disease or carrier asymptomatic states in which bacteria persist in intracellular locations. Specifically, bacterial infections which become innately resistant to antibiotics, preferably beta-lactams when it naturally adapts to the hostile intraphagosomal environment inside eukaryotic cells.

In a preferred embodiment, the condition treated are systemic infections caused by bacteria harbouring the INDAR genes. In a more preferred embodiment, the conditions treated are selected from the list consisting of: salmonellosis, typhoid fever, non-typhoid fever, gastroenteritis, arthritis, polymicrobial infections, respiratory infections, systemic infections and asymptomatic intracellular persistent and chronic infections.

Another aspect of the present invention relates to a method *in vitro* to identify INDAR inhibitors, hereinafter the screening method of the invention, wherein the method comprises:
a) Contacting a candidate compound with a bacterial culture of pathogenic bacteria, wherein the pathogenic bacteria comprises a non-functional gene encoding for a PBP protein selected from the list consisting of: a PBP protein having at least 80% identity with SEQ ID NO: 6 and/or a PBP protein having at least 80% identity with SEQ ID NO: 8,
b) Incubate the candidate compound with the bacterial culture of step a) in a medium with acidic pH range from 4.0 to 5.0;
c) Comparing the growth of bacterial culture of step b) with the growth of the corresponding pathogenic wild-type bacterial culture in the presence of the candidate compound;
wherein a decreased growth of the pathogenic bacterial culture comprises the non-functional gene encoding for a PBP protein selected from the list consisting of: a PBP protein having at least 80% identity with SEQ ID NO: 6 and/or a PBP protein having at least 80% identity with SEQ ID NO: 8, regarding the growth of corresponding pathogenic wild-type bacterial culture, indicates that the candidate compound is an INDAR inhibitor.

In general, the aforementioned screening method as well as INDAR inhibitors identified therefrom have applicability as medicament, preferably as antibiotic in relation to the treatment and/or prevention of intracellular bacterial infections and opportunistic and polymicrobial intracellular infections mentioned herein.

The term "non-functional gene" refers to a gene that does not express the expected functional polypeptide, e.g. does not allow the production of the expected active enzyme. In some embodiments, the said gene is not expressed in the recombinant strain, e.g. the said gene is not transcribed or the corresponding transcription product is not translated into a protein, e.g. into an enzyme, having its usual activity. In another embodiment, the coding sequence is partly or completed deleted. In another embodiment, the promoter sequence is deleted. In another embodiment, the gene is inactivated, in particular by introduction of an insert into the coding sequence of said gene. In another embodiment, the non-functional gene can be a mutant gene.

The term "PBP" as used herein refers to "Penicillin-binding protein", which are a group of bacterial proteins that bind to penicillin and other antibiotics of the β-lactam antibiotic family. Penicillin-binding proteins are generally enzymes involved in peptidoglycan biosynthesis, so play essential roles in bacterial cell wall biosynthesis. PBPs bind beta-lactam antibiotics because the chemical structure of these antibiotics is similar to the dipeptide D-Alanine-D-Alanine (D-Ala-D-Ala) present at the end of the stem peptides incorporated into the peptidoglycan as part of a sugar-peptide precursor molecule (muropeptide).

The term "PBP2" (Penicillin Binding Protein 2) also called in the literature Peptidoglycan D,D-transpeptidase MrdA. In the context of the present invention, PBP2 is also defined by a nucleotide or polynucleotide sequence, which constitutes the coding sequence of the PBP2 protein, and which would comprise various variants from:
a) nucleic acid molecules encoding a polypeptide comprising the amino acid sequence of SEQ ID NO: 6,
b) nucleic acid molecules whose chain complement it hybrid with the polynucleotide sequence of a),
c) nucleic acid molecules whose sequence differs from a) and/or b) due to the degeneracy of the genetic code,
d) nucleic acid molecules encoding a polypeptide comprising the amino acid sequence with an identity of at least 80%, 85%, 90%, 91%, 92%, 93%, 94%, 95%, 96%, 97%, 98% or 99% with SEQ ID NO: 6. in that the polypeptide encoded by said nucleic acids possesses the activity and structural characteristics of the PBP2 protein. Among these nucleic acid molecules is the SEQ ID NO: 5.

In another particular embodiment, the PBP2 comprises a nucleotide sequence having a sequence identity of, at least, 100% with the sequence SEQ ID NO: 5. In a most particular embodiment, the PBP2 consists of the nucleotide sequence SEQ ID NO: 5. In another particular embodiment, PBP2 comprises an amino acid sequence having a sequence identity of, at least, 100% with the sequence SEQ ID NO: 6. In a most particular embodiment, the PBP2 consists of the amino acid sequence SEQ ID NO: 6.

The term "PBP3" (Penicillin Binding Protein 3) also called in the literature Peptidoglycan D,D-transpeptidase Ftsl. In the context of the present invention, PBP3 is also defined by a nucleotide or polynucleotide sequence, which constitutes the coding sequence of the PBP3 protein, and which would comprise various variants from:
a) nucleic acid molecules encoding a polypeptide comprising the amino acid sequence of SEQ ID NO: 8,
b) nucleic acid molecules whose chain complement it hybrid with the polynucleotide sequence of a),
c) nucleic acid molecules whose sequence differs from a) and/or b) due to the degeneracy of the genetic code,
d) nucleic acid molecules encoding a polypeptide comprising the amino acid sequence with an identity of at least 80%, 85%, 90%, 91%, 92%, 93%, 94%, 95%, 96%, 97%, 98% or 99% with SEQ ID NO: 8. in that the polypeptide encoded by said nucleic acids possesses the activity and structural characteristics of the PBP3 protein. Among these nucleic acid molecules is the SEQ ID NO: 7.

In another particular embodiment, the PBP3 comprises a nucleotide sequence having a sequence identity of, at least, 100% with the sequence SEQ ID NO: 7. In a most particular embodiment, the PBP3 consists of the nucleotide sequence SEQ ID NO: 7. In another particular embodiment, PBP3 comprises an amino acid sequence having a sequence identity of, at least, 100% with the sequence SEQ ID NO: 8. In a most particular embodiment, the PBP3 consists of the amino acid sequence SEQ ID NO: 8.

In a preferred embodiment of the screening method of the invention, the non-functional gene of the pathogenic bacteria of step (a) is the PBP2 and/or the PBP3. In a more preferred embodiment, the PBP2 gene comprises the SEQ ID NO: 5, and the PBP3 gene comprises the SEQ ID NO: 7.

In another preferred embodiment the acidic pH of step (b) is preferably between 4.3 to 5, more preferably is a pH selected from the list consisting of 4.4, 4.5, 4.6, 4.7, 4.8 and 4.9, more preferably the acidic pH is 4.6.

In another aspect, the present invention provides INDAR inhibitors according to the present invention, obtainable by the screening method disclosed herein.

In another aspect, the present invention provides a mutant bacteria cell, which does not express the polynucleotide sequence encoding the polypeptide selected from the list consisting of: PBP2 (SEQ ID NO: 6), PBP2SAL (SEQ ID NO: 2), PBP3 (SEQ ID NO: 8), PBP3SAL (SEQ ID NO: 4) or any combinations thereof.

In a more preferred embodiment, the mutant bacteria cell of the present invention is a Gram-negative bacteria (as described in the present disclosure), more preferably a bacteria selected from the list consisting of: *Escherichia spp., Salmonella spp., Citrobacter spp., Klebsiella spp., Enterobacter spp., Lelliottia spp., Cedecea spp., Raoultella spp., Erwinia spp., Leclercia spp., Kluyvera spp., Mixta* spp., *Pantoea spp, Pluribacter spp., Rosenbergiella spp., Morganella spp., Tatumella spp., Serratia spp., Acinetobacter spp.,* and *Haemophilus spp.* In a more preferred embodiment, the mutant bacteria cell of the present invention belongs to the *Salmonella spp.,* preferably is the species *S*. *enterica,* more preferably the *subspecies I* (*enterica*) and more preferably, the serovar Typhimurium.

In a preferred embodiment, the mutant bacteria cell of the present invention belongs to the species *S*. Typhimurium SV5015. In a more preferred embodiment, the mutant *S*. Typhimurium SV5015 cells of the present invention are selected from the list consisting of:
- S. Typhimurium SV5015 which does not express the polynucleotide sequence encoding the PBP3 polypeptide (SEQ ID NO: 8). In the present disclosure, this mutant bacterial cell is named as MD4356 (ΔPBP3),
- *S*. Typhimurium SV5015 which does not express the polynucleotide sequence encoding the PBP3SAL polypeptide (SEQ ID NO: 4). In the present disclosure, this mutant bacterial cell is named as MD2577 *(*Δ*PBP3SAL*),
- *S*. Typhimurium SV5015 which does not express the polynucleotide sequence encoding the PBP2 polypeptide (SEQ ID NO: 6). In the present disclosure, this mutant bacterial cell is named as MD5052 *(*Δ*PBP2*),
- *S*. Typhimurium SV5015 which does not express the polynucleotide sequence encoding the PBP2SAL polypeptide (SEQ ID NO: 2). In the present disclosure, this mutant bacterial cell is named as MD2576 (Δ*PBP2SAL*),
- *S*. Typhimurium MD5052 in which the PBP2SAL gene (SEQ ID NO. 1) was replaced in its native chromosomal location by the PBP2 gene (SEQ ID NO: 5). In the present disclosure, this mutant bacterial cell is named as MD5544 (Δ*mrdA* Δ*PBP2SAL pPBP2SAL*::*mrdA),*
- *S*. Typhimurium MD4356 in which the PBP3SAL gene (SEQ ID NO. 3) was replaced in its native chromosomal location by the PBP3 gene (SEQ ID NO: 7). In the present disclosure, this mutant bacterial cell is named as MD5542 (Δ*ftsI* Δ*PBP3SAL pPBP3SAL*::*ftsI)*,
- *S*. Typhimurium MD5061 which does not express the polynucleotide sequence encoding the PBP2 polypeptide (SEQ ID NO: 6) and the PBP3 polypeptide (SEQ ID NO: 8). In the present disclosure, this mutant bacterial cell is named as MD5063 (ΔftsI ΔmrdA = ΔPBP2 ΔPBP3), and
- *S*. Typhimurium MD2580 which does not express the polynucleotide sequence encoding the PBP2SAL polypeptide (SEQ ID NO: 2) and the PBP3SAL polypeptide (SEQ ID NO: 4). In the present disclosure, this mutant bacterial cell is named as MD2588 (Δ*PBP2SAL* Δ*PBP3SAL).*

In another aspect, the present invention relates to the use of a pathogenic bacterial cell comprising a non-functional gene encoding for a PBP, or a mutant gene encoding for a PBP, according to the present invention, and other enzymes involved in the synthesis, hydrolysis and remodelling of the peptidoglycan for the *in vitro* identification and/or validation of INDAR inhibitors.

In another aspect, the present invention relates to a pharmaceutical composition comprising a therapeutically effective amount of the INDAR inhibitor according to the present invention, and at least one or more adjuvants and/or pharmaceutically acceptable vehicles, useful for the treatment and/or prevention of intracellular bacterial infections caused by bacteria harbouring the INDAR genes. In a more preferred embodiment, the conditions treated are selected from the list consisting of: salmonellosis, typhoid fever, non-typhoid fever, gastroenteritis, arthritis, polymicrobial infections, respiratory infections, systemic infections and asymptomatic intracellular persistent and chronic infections.

The adjuvants and pharmaceutically acceptable vehicles that can be used in said compositions are the adjuvants and vehicles known by experts in the art and commonly used in the production of therapeutic compositions. The term "pharmaceutically acceptable vehicle" is a substance used in the composition to dilute any of the components comprised therein up to a certain volume or weight. The pharmaceutically acceptable vehicle is an inert substance or of identical action to any of the elements comprised in the composition of the present invention. The function of the vehicle is to facilitate the incorporation of other elements, allow better dosing and administration or give the composition consistency and form. Additionally, the pharmaceutical composition of the invention may comprise one or more adjuvants and/or excipients. The term "excipient" makes reference to a substance that aids the absorption of the elements of the composition of the invention, stabilizes said elements, activates or aids the preparation of the composition in the sense of giving it consistency or providing flavors that make it more pleasant. Thus, the excipients could have the function of maintaining the ingredients bound together, such as for example in the case of starches, sugars or celluloses, the function of sweetening, the function of coloring, the function of protecting the composition, such as for example, to insulate it from air and/or humidity, the function of filling a tablet, capsule or any other form of presentation, a disintegratory function for facilitating the dissolution of the components and its absorption in the intestine, without excluding other types of excipients not mentioned in this paragraph.

In the sense used in the description, the expression "therapeutically effective amount" refers to the amount of the INDAR inhibitor according to the present invention, calculated to produce the required effect and, in general, will be determined, among other factors, by the inherent properties of the compounds, including the age, condition of the patient, severity of the alteration or disorder, and the route and frequency of administration.

In a particular embodiment, said therapeutic composition is prepared in solid form or in aqueous suspension, in a pharmaceutically acceptable diluent. The therapeutic composition provided by this invention may be administered via any appropriate route of administration, wherefore said composition shall be formulated in the suitable pharmaceutical form for the selected route of administration. In a particular embodiment, administration of the therapeutic composition provided by the invention is carried out parenterally, orally, intraperitoneally, subcutaneously, intracranial, etc. A revision of the different pharmaceutical forms of drug administration and the excipients required to obtain them can be found, for example, in the "Treaty of Galenic Pharmacy", C. Faulii Trillo, 1993, Luzán 5, S.A. Ediciones, Madrid.

The therapeutic compositions of the invention can be administered in combination with other drugs used in the treatment of intracellular bacterial infections caused by bacteria harbouring the INDAR genes, preferably conditions selected from the list consisting of: salmonellosis, typhoid fever, non-typhoid fever, gastroenteritis, arthritis, polymicrobial infections, respiratory infections, systemic infections and asymptomatic intracellular persistent and chronic infections, with a view to acting in a complementary manner or as reinforcement.

In another aspect, the present invention relates to a method of treating and/or preventing intracellular bacterial infections, intracellular asymptomatic infections, and intracellular polymicrobial and opportunistic infections, according to the present invention, comprising administering in a therapeutically or prophylactically effective amount of an INDAR inhibitor or a pharmaceutical composition of the invention, to a subject in need of such treatment.

As used herein, the term "subject" refers to human and non-human animals, such as veterinary subjects. The term "non-human animal" includes all vertebrates, e.g., mammals and non-mammals, such as non-human primates such as chimpanzees and other apes and monkey species; farm animals such as cattle, sheep, pigs, goats, and horses; domestic mammals such as dogs and cats; laboratory animals including rodents such as mice, rats, rabbits and guinea pigs, and the like. In a particular embodiment, the subject is a human, man or woman of any age or race. In another embodiment, the term subject also refers to avian. The term "avian" as used herein, refers to any bird of any avian species, but are primarily intended to encompass poultry which are commercially raised for eggs, meat or as pets. Accordingly, the terms "avian" and "avian subject" are particularly intended to encompass various birds including, but not limited to, chickens, turkeys, ducks, geese, quail, pheasant, parakeets, parrots, cockatoo, cockatiel, ostrich, emu, etc.

The subject object of the present invention is characterized by comprising an intracellular bacterial infection, an asymptomatic intracellular bacterial infection, or an intracellular polymicrobial and opportunistic bacterial infection according to the present invention.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Fig.1** **Substitution of PBP2 by PBP2SAL and PBP3 by PBP3SAL in S. Typhimurium strain SL1344 grown in minimal nutrient medium PCN at pH 4.6. (A)** Relative levels of the four PBPs in bacteria grown in PCN minimal medium at the indicated pH values. **(B)** Kinetics of the replacement of PBP2 by PBP2SAL and PBP3 by PBP3SAL as bacteria were shifted from PCN pH 7.4 to PCN pH 4.6 medium (time 0). Shown are the respective Western blots and the Coomassie staining as proof of similar loading among samples.
**Fig. 2****. (A)** Functional domain organization and extent of identity between PBP2SAL and PBP2 from S. Typhimurium and PBP2 of *Escherichia coli.* TM, transmembrane domain. SXXK, SXN/D and KSG indicate the catalytic motifs. The PBP dimer (now known as SEDS-interacting domain) and transpeptidase functional domains (Pfam no. PF03717 and PF00905, respectively) are also depicted. **(B)** region of the S. Typhimurium genome (strain SL1344) in which the gene encoding PBP2SAL (*SL1344_1845* = *STM1910*) is inserted. For comparison, the same region in the *E*. *coli* genome (strain MG1655), is shown.
**Fig. 3****.** Distribution of *S*. Typhimurium PBP2SAL **(A)** and PBP3SAL **(B)** homologs in bacterial genera. BLASTp analyses were performed using PBP2SAL and PBP3SAL protein sequences from S. Typhimurium strain SL1344 as queries and excluding the *Salmonella* genus. Hits with highest score and having more than 80% identity and 95% coverage were subjected to the taxonomy tool of NCBI with the method FNM, a max sequence difference of 0.8 and Grishin distance (protein).

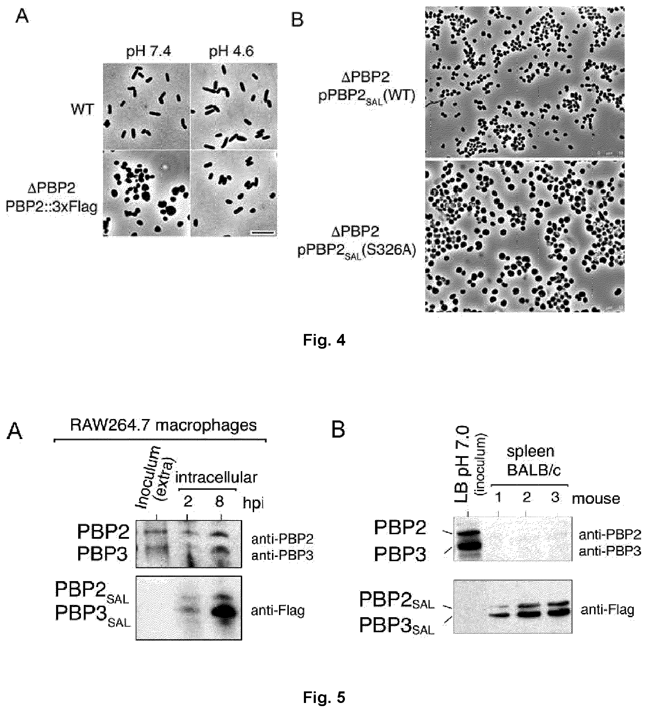

**Fig. 4****.** The 3xFlag epitope-tagged PBP2SAL variant of S. Typhimurium retains function in cell elongation and it has the conserved serine at position 326 (S326) as critical residue for catalysis. **(A)** PBP2SAL-3xFlag-mediated restoration of rod-cell morphology in a S. Typhimurium ΔPBP2 mutant occur only at acid pH (4.6). **(B)** a catalytically-death variant of PBP2SAL (S326A) cannot restore cell morphology in a S. Typhimurium ΔPBP2 mutant. Bar, 5 µm.
**Fig. 5****.** S. Typhimurium PBP2SAL and PBP3SAL prevail over PBP2 and PBP3 in intracellular bacteria and *in vivo* in host target organs. **(A)** Production of PBP2SAL and PBP3SAL in bacteria located inside cultured RAW267.4 mouse macrophages, hpi, hours post infection. **(B)** PBP2SAL and PBP3SAL prevail over PBP2/PBP3 in bacteria colonizing the spleen during systemic infection of BALB/c mice. *In vivo* data (spleen extracts) were obtained from there independent mice numbered 1, 2, and 3.

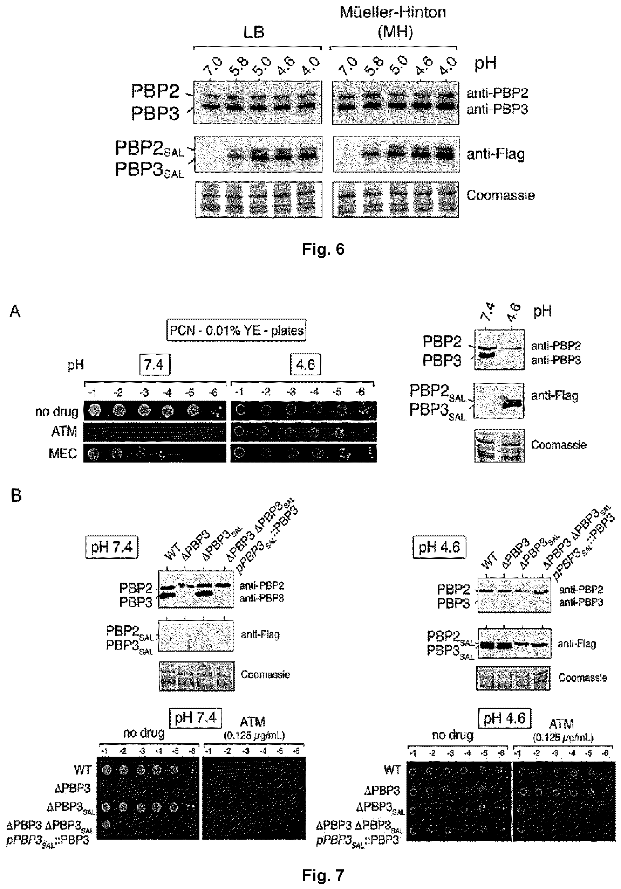

**Fig. 6****.** Nutrient rich media do not recapitulate the replacement of PBP2 and PBP3 by PBP2SAL and PBP3SAL. Shown are the relative levels of PBP2, PBP3, PBP2SAL and PBP3SAL in S. Typhimurium strain SL1344 growing in LB and Mueller-Hinton media at distinct pH values.
**Fig. 7****.** Growth of S. Typhimurium in acidified medium limited in nutrients decreases susceptibility to beta-lactams. **(A)** Reduced susceptibility of S. Typhimurium to mecillinam (MEC) (5 µg/mL) and aztreonam (ATM) (0.125 µg/mL) in PCN pH 4.6-0.01% yeast extract medium. **(B)** PBP3SAL is responsible for the increased resistance to ATM displayed by S. Typhimurium in PCN pH 4.6-0.01% YE plates. Western blots show PBP2, PBP3, PBP2SAL and PBP3SAL relative levels in the isogenic mutant strains analysed. The strain ΔPBP3ΔPBP3SAL-pPBP3SAL::PBP3 expresses PBP3 from the acid-responsive promoter of the gene encoding PBP3SAL, a genetic construction made in the chromosome following replacement of PBP3SAL coding sequence by that of PBP3.

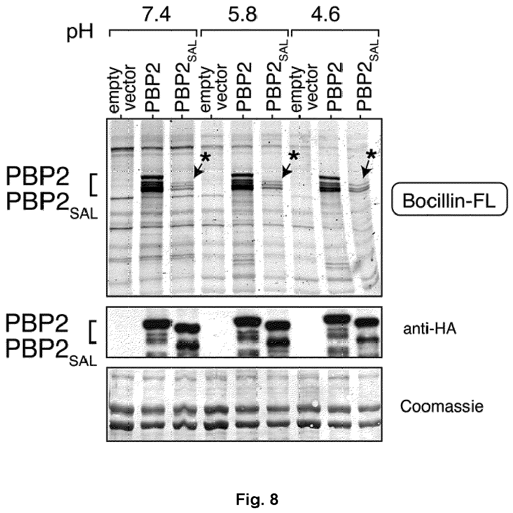

**Fig. 8****.** PBP2SAL binds beta-lactams with less affinity than PBP2. Fluorescence-based assay showing Bocillin-FL binding to membrane extracts from *E. coli* strain SP4500 *[mrdA(ts)]* harboring empty vector or vectors expressing HA-tagged variants of PBP2 and PBP2SAL from S. Typhimurium. Indicated are the pH values at which the binding assay was performed. The asterisk points to the signal obtained for PBP2SAL.

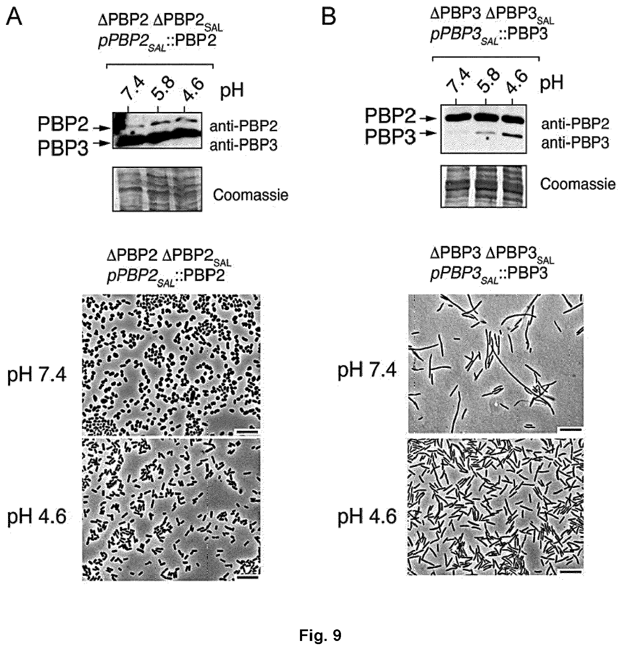

**Fig. 9****.** Response to acid pH of S. Typhimurium recombinant strains in which the genes expressing PBP2SAL or PBP3SAL were replaced in their native chromosome locations by the coding sequences of PBP2 or PBP3. Previous to this modification, the strains were knocked-out for the respective genes encoding PBP2 or PBP3. **(A)** expression of PBP2 under the promoter of PBP2SAL at acidic pH correlates with restoration of rod morphology. **(B)** expression of PBP3 under the promoter of PBP3SAL at acid pH restores cell division.

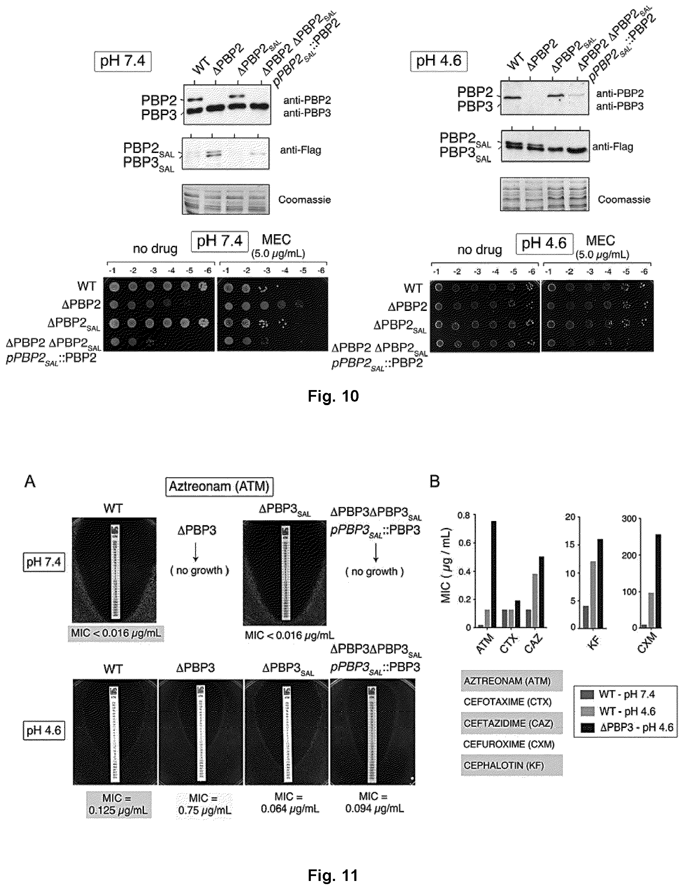

**Fig. 10****.** Susceptibility to mecillinam (MEC) at pH 4.6 and 7.4 of S. Typhimurium isogenic strains lacking PBP2, PBP2SAL or expressing PBP2 from the promoter of the gene encoding PBP2SAL. The strains were also analyzed to determine relative levels of the four PBPs of interest.
**Fig. 11****.** PBP3SAL promotes beta-lactam resistance in S. Typhimurium. **(A)** E-test assays show a marked increase in the MIC value to ATM in response to acid pH (4.6) that is further enhanced in the ΔPBP3 mutant, expressing only PBP3SAL as main enzyme involved in cell division. **(B)** MIC values obtained by the E-test assay for different beta-lactams that bind with high affinity to PBP3: aztreonam (ATM), cefotaxime (CTX), ceftazidime (CAZ), cefuroxime (CXM) and cephalotin (KF).

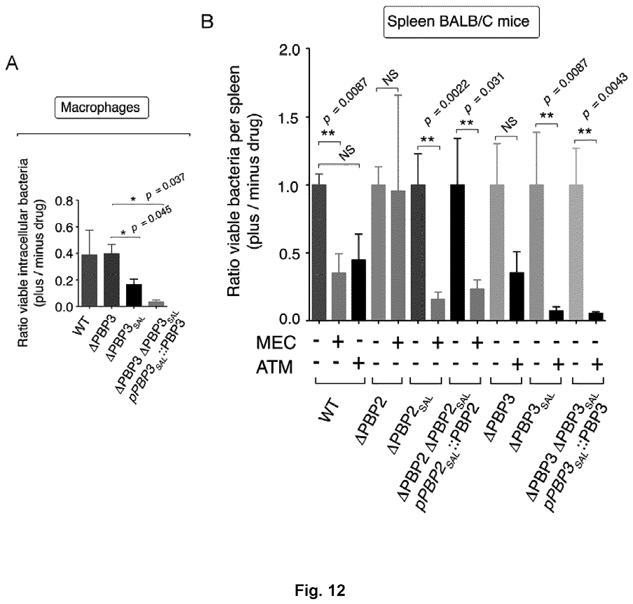

**Fig. 12****.** PBP2SAL and PBP3SAL confers antibiotic resistance in the animal host. **A)** Susceptibility to aztreonam (ATM) (10 µg/mL) in bacteria located inside macrophages. **B)** In vivo effect of MEC and ATM in bacterial viability following intraperitoneal challenge of BALB/c mice. MEC and ATM were administrated every 24 h during 3 days at doses of 20 mg and 10 mg per mouse, respectively. Shown are the individual data points, means and standard deviations (SD) from three biological replicates (macrophages, panel a) and minimum of four BALB/c mice (n ≥4).

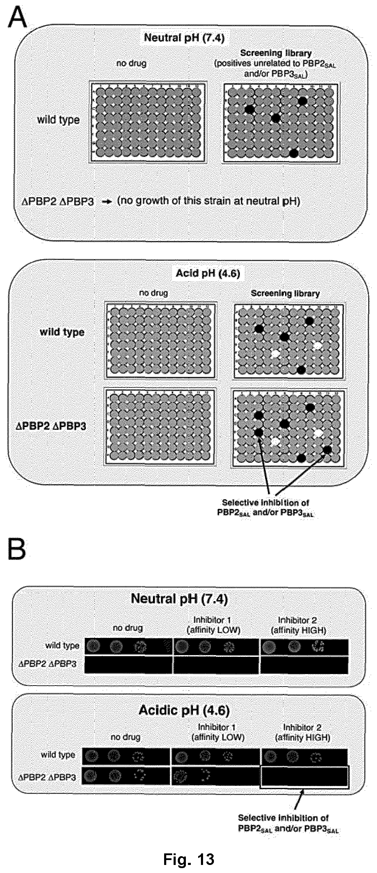

**Fig. 13****. Basis of the screening method to select for inhibitors that specifically inactivate the INDAR proteins PBP2SAL and/or PBP3SAL (A)** A chemical library deposited in a multiwall plate is tested for growth inhibition of isogenic wildtype and mutant defective in PBP2 and PBP3. This ΔPBP2ΔPBP3 mutant is unable to grow a neutral pH since PBP2SAL and PBP3SAL support growth only at acidic pH. Note that the compounds that abolish growth of the mutant, but not the wild-type, at acidic pH are candidate to act as INDAR inhibitors. **(B)** Examples of two chemicals (inhibitors 1 and 2) abolishing growth of ΔPBP2ΔPBP3 double mutant at acidic pH having distinct affinity for the target(s).

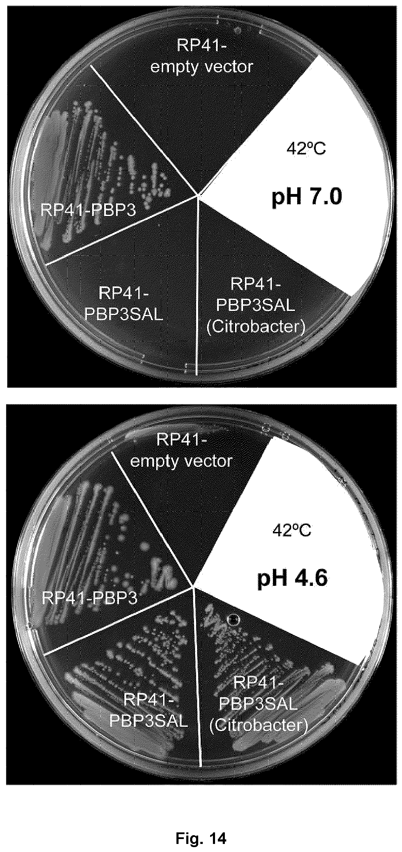

**Fig. 14****. The PBP3SAL ortholog present in *Citrobacter freundii* is functional only at acidic pH.** Shown are plates with different derivates of *E. coli* strain RP41, carrying a thermosensitive mutation in PBP3 that makes bacteria unable to divide at 42° C (Table 1). The different strains harbour plasmids expressing no protein (empty vector), PBP3 from *S*. Typhimurium (RP41-PBP3), PBP3SAL from *S*. Typhimurium (RP41-PBP3SAL) or the PBP3SAL orthlog present in *C. freundii.* Note that the two PBP3SAL proteins tested restore cell division and growth only in acidic pH.

### Examples

### MATERIALS AND METHODS

### Media, bacterial strains, and plasmids.

**Table 1. S. Typhimurium and E. coli strains and plasmids used in the present invention for construction of new strains used in the examples.**

| Bacterial strain/ plasmid | Relevant genotype | Source / Reference |
|---|---|---|
| ***Escherichia coli*** | | |
| MC6RP1 | K-12, F⁻, *thrA leuA proA dra drm lysA* | García-del Portillo, F. & de Pedro,M. A. J. Bacteriol., 1990; 172: 5863-5870 |
| RP41 | M6RP1, *leu+, ftsI* (ts) | García-del Portillo, F. & de Pedro,M. A. J. Bacteriol., 1990; 172: 5863-5870 |
| SP4500 | K-12 F⁻ *his pro purB thi mtl xyl galK lacY rpsL pbpA45*(Ts) | García-del Portillo, F. & de Pedro,M. A. J. Bacteriol., 1990; 172: 5863-5870 |
| MD5005 | RP41, pAC-6xHIS | Castanheira, S. et al. MBio. 2017; 8, doi: 10.1128/mBio.01685-17 |
| MD5006 | RP41, pAC-6xHIS-*STM1836 (PBP3SAL)* | Castanheira, S. et al. MBio. 2017; 8, doi: 10.1128/mBio.01685-17 |
| MD5008 | RP41, pAC-6xHIS-*ftsI* (*PBP3*) | Castanheira, S. et al. MBio. 2017; 8, doi: 10.1128/mBio.01685-17 |

| ***S*. Typhimurium** | | |
|---|---|---|
| Bacterial strain/ plasmid | Relevant genotype | Source / Reference |
| SV5015 | SL1344, *his*G⁺ | Vivero, A. et al. Journal of Bacteriology 2008; 190: 1152-1156. |
| MD2502 | SV5015 ΔSTM1836-Kan | Castanheira, S. et al. MBio. 2017; 8, doi: 10.1128/mBio.01685-17 |
| MD2577 | SV5015 Δ*STM1836* | Castanheira, S. et al. MBio. 2017; 8, doi: 10.1128/mBio.01685-17 |
| MD4356 | SV5015 Δ*ftsI* | Castanheira, S. et al. MBio. 2017; 8, doi: 10.1128/mBio.01685-17 |
| MD2559 | SV5015 *STM1836*::3xFLAG-kan | Castanheira, S. et al. MBio. 2017; 8, doi: 10.1128/mBio.01685-17 |

| **Plasmids** | | |
|---|---|---|
| pCP20 | FLP⁺, Amp^{R}, Cm^{R} | Cherepanov, P. P. & Wackernagel, W. Gene. 1995; 158: 9-14. |
| pKD13 | Kan^{R}, Amp^{R} | Datsenko, K. A. & Wanner, B. L. Proc Natl Acad Sci U S A. 2000; 97: 6640-6645 |
| pKD46 | γ, β, exo. Amp^{R} | Datsenko, K. A. & Wanner, B. L. Proc Natl Acad Sci U S A. 2000; 97: 6640-6645 |
| pAC-Plac | Cm^{R} | Castanheira, S. et al. MBio. 2017; 8, doi: 10.1128/mBio.01685-17 |

| Bacterial strain/ plasmid | Relevant genotype | Source / Reference |
|---|---|---|
| pAC-6xHIS | Cm^{R} | Lobato-Marquez, D., et al. Scientific Reports 2015; 5: 9374 |
| pFUS-HA-P_{Ara} | Kan^{R} | Lobato-Marquez, D., et al. Scientific Reports 2015; 5: 9374 |

**Table 2. Oligonucleotides used in this invention for construction of new bacterial mutant strains.**

| **Name** | **Sequence (5'-3')** | **SEQ ID NO.** |
|---|---|---|
| KO STPBP2 FW | CAACCACCGGGTTTTCCGCAGGCAAATGGGTGTT GTTGTCGCCCAGCATGGTGTAGGCTGGAGCTGCT TC | 11 |
| KO STPBP2 RV | ATGAAACGACAAAATTCTTTTCGTGACTATACGGC TGAGTCCGCACTGTTATTCCGGGGATCCGTCGAC C | 12 |
| pbp2 flanking FW | GGATCGATATGAATTTTATCCCAGA | 13 |
| pbp2 flanking RV | CGGGCGTGGAGCATCACAACCAACC | 14 |
| PBP2SAL S326A FW | AAGGGCTTTATCCTCCGGCGGCAACGGTTAAACC CTATAT | 15 |
| PBP2SAL S326A RV | ATATAGGGTTTAACCGTTGCCGCCGGAGGATAAA GCCCTT | 16 |
| Exchange PBP3SAL by PBP3 1 | CCGTTTCCCGTTAAATCAATCACCTGAAAAATGATT CGGCTGGAGATCAGATGAAAGCAGCGGCAAAAAC | 17 |
| Exchange PBP3SAL by PBP3 2 | GAAGCAGCTCCAGCCTACACTTACGATCTGCCACC TGTTC | 18 |
| Exchange PBP3SAL by PBP3 3 | GAACAGGTGGCAGATCGTAAGTGTAGGCTGGAGC TGCTTC | 19 |
| Exchange PBP3SAL by PBP3 4 | GGGCGCTAAAGGGCGCAAGTGTAACGCGAATTGC GCCCCGGGAAAATCCTATTCCGGGGATCCGTCGA CC | 20 |
| Exchange PBP2SAL by PBP2 1 | GGCGCTGACCGCCCCGTATCATTAGGCGGCGGAT TGTTCAGGTACAGGTAATGAAACGACAAAATTCTT TTCGTG | 21 |
| Exchange PBP2SAL by PBP2 2 | GAAGCAGCTCCAGCCTACACTTATTGGTCCTCCG CCGCTGCAACC | 22 |
| Exchange PBP2SAL by PBP2 3 | GGTTGCAGCGGCGGAGGACCAATAAGTGTAGGCT GGAGCTGCTTC | 23 |
| Exchange PBP2SAL by PBP2 4 | ATGCAGATATTCCGGCCGTATCCTTGTCTGATGGC GCTTTGCTTATTTGAATTCCGGGGATCCGTCGACC | 24 |
| fwSpeI-PBP2 | CGCTACTAGTATGAAACGACAAAATTCTTTTCGTG | 25 |
| revSpe-PBP2 | CCCTACTAGTTTATTGGTCCTCCGCCGCTGCAACC | 26 |
| fwSpeI-PBP2* | CGCTACTAGTATGACTTTTAAAGACTTTGATGCGG | 27 |
| revSpeI-PBP2* | CCCTACTAGTCTATGGCGCCTGATCCGGCTGTGT G | 28 |
| pAC FW | CGGCCCTCATTCGTGCGCTCTAGGA | 29 |
| pAC-sec | CATAATGGGGAAGGCCATCCAG | 30 |
| pFUS FW | GCACGGCGTCACACTTTGCTATGCC | 31 |
| pFUS REV | GCTAAGATCTCCCATATGTATATCT | 32 |
| FLAG-2*-Fw | TGATCCACAGGCTGATACCACACAGCCGGATCAG GCGCCAGACTACAAAGACCATGACGG | 33 |
| FLAG-2*-Rv | TCCGGCCGTATCCTTGTCTGATGGCGCTTTGCTTA TTTGACATATGAATATCCTCCTTAG | 34 |
| fwSpeI-PBP3Citro | CGCTACTAGTGTGAAAAAGAAAAACGACGGCGAC G | 35 |
| revPvuI-PBP3Citro | CCCTCGATCGTTACAGCGACGGCGAGGATACGTT G | 36 |
| FL-2* Fw | AGGCCTGACTCCGGATAACGGA | 37 |
| FL-2* Rv | ACCTCTATGCCTTACGGGCAG GGCGCTGACCGCCCCGTATCATTAGGCGGCGGAT | 38 |
| KO PBP2* Fw | TGTTCAGGTACAGGTAGTGTAGGCTGGAGCTGCT TC | 39 |
| KO PBP2* Rv | ATGCAGATATTCCGGCCGTATCCTTGTCTGATGGC GCTTTGCTTATTTGAATTCCGGGGATCCGTCGACC | 40 |
| FL-pbp2*-FW | AGGCCTGACTCCGGATAACGGA | 41 |
| FL-pbp2*-RV | ACCTCTATGCCTTACGGGCAG | 42 |

### E. coli and S. Typhimurium strains and plasmids constructed for use in the examples.

**MD5051.** Bacterial strain derivate of *E*. *coli* SP4500 (**Table 1**) transformed with plasmid pFUS-HA **(Table 1).**

**MD5053.** Bacterial strain derivate of *E. coli* SP4500 **(Table 1)** transformed with plasmid pFUS-HA::*mrdA*. To construct pFUS-HA::*mrdA* plasmid, the *PBP2* gene (SEQ ID NO. 5) was amplified by PCR from genomic DNA of *S*. Typhimurium strain SL1344 using the oligonucleotides fwSpeI-PBP2/revSpe-PBP2 (SEQ ID NO: 25/SEQ ID NO: 26) **(Table 2)**, digested with Spel and ligated into the vector plasmid pFUS-HA-Para (Lobato-Marquez, D., et al. Sci Rep. 2015; 5: 9374). The final construct was sequenced to ensure the absence of undesired mutations.

**MD5040.** Bacterial strain derivate of *E*. *coli* SP4500 **(Table 1)** transformed with plasmid pFUS-HA::*STM1910*. To construct pFUS-HA::*STM1910* plasmid, the *PBP2SAL* gene (SEQ ID NO.1) was amplified by PCR from genomic DNA of *S*. Typhimurium strain SL1344 using the oligonucleotides fwSpeI-PBP2*/revSpeI-PBP2* (SEQ ID NO: 27/SEQ ID NO: 28) **(Table 2)**, digested with Spel and ligated into the vector plasmid pFUS-HA-Para (Lobato-Marquez, D., et al. Sci Rep. 2015; 5: 9374). The final construct was sequenced to ensure the absence of undesired mutations.

**MD5052.** Bacterial strain derivate of *S*. Typhimurium SV5015 **(Table 1)** defective in *mrdA* (Δ*mrdA*). To delete the native copy of *mrdA (PBP2* gene*)* (SEQ ID NO. 5), a KanR cassette was used containing 50 bp upstream and downstream flanking regions of *PBP2* coding sequence. A DNA fragment containing KanR cassette and flanking sequences was amplified by PCR from template plasmid pKD13 (Datsenko, K. A. & Wanner, B. L. Proc Natl Acad Sci U S A. 2000; 97: 6640-6645) using primers KO STPBP2 FW/KO STPBP2 RV (SEQ ID NO: 11/SEQ ID NO: 12) **(Table 2).** Gene replacement in the chromosome was done using Lambda Red-mediated recombination, as described (Ellermeier, C. D., et al. Gene. 2002; 290: 153-161). After electroporation, Super Optimal Broth with Catabolite repression (SOC) medium buffered at pH 4.6 with MES [2-(N-morpholino) ethanesulfonic acid] (69892, Sigma-Aldrich) was added and cells were grown for 3h. Recombinants were selected on LB agar pH 4.6 supplemented with 60 µg/ml kanamycin. Oligonucleotides pbp2-flanking-FW and pbp2-flanking-RV (SEQ ID NO: 13/SEQ ID NO: 14) **(Table 2)** were used to confirm the gene deletion. All final constructions were verified by sequencing.

**MD2576.** Bacterial strain derivate of S. Typhimurium SV5015 **(Table 1)** defective in *STM1910* (Δ*STM1910 =* Δ*PBP2SAL*). To delete the native copy of *STM1910 (PBP2SAL* gene) (SEQ ID NO. 1) a KanR cassette was used containing upstream and downstream regions (50 bp) of the *PBP2SAL* coding sequence. The KanR cassette was amplified by PCR from plasmid pKD13 (Datsenko, K. A. & Wanner, B. L. Proc Natl Acad Sci U S A. 2000; 97: 6640-6645) using primers KO PBP2* FW/ KO PBP2* RV (SEQ ID NO: 39/SEQ ID NO: 40) **(Table 2).** Gene replacement at the chromosome was done using Lambda Red-mediated recombination, as described (Ellermeier, C. D., et al. Gene. 2002; 290: 153-161). Oligonucleotides FL-pbp2*-FW/FL-pbp2*-RV (SEQ ID NO: 41/SEQ ID NO: 42) **(Table 2)** were used to confirm the gene deletion. All final constructions were verified by sequencing.

**MD2540.** Bacterial strain derivate de S. Typhimurium SV5015 **(Table 1)** in which a 3xFLAG epitope was added to the 3' end of the coding sequence of the *PBP2SAL* gene (SEQ ID NO. 1) in this native chromosomal location using the method of Uzzau et al. (Proc. Natl. Acad. Sci. USA 97:6640.5, 2000) and the oligonucleotides FLAG-2*-Fw and FLAG-2*-Rv (SEQ ID NO: 33/SEQ ID NO: 34) **(Table 2). Genotype:** *PBP2SAL*::3xFLAG-Kan

**MD5054.** Bacterial strain derivate of *S*. Typhimurium MD2540 in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** *PBP2SAL*::3xFLAG.

**MD5056.** Bacterial strain derivate of S. Typhimurium MD5054 in which the *PBP3SAL*::3xFLAG-Kan allele from donor strain MD2559 **(Table 1)** was introduced by generalized transduction using the bacteriophage P22 H105/1 *int201* (Schmieger, Molecular General Genetics 1972, 119: 75-88). **Genotype:** *PBP2SAL*::3xFLAG *PBP3SAL*::3xFLAG-Kan.

**MD5064.** Bacterial strain derivate of *S*. Typhimurium MD5056 in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** SV5015 *PBP2SAL*::3xFLAG.

**MD5060:** Bacterial strain derivate of S. Typhimurium MD5052 (Δ*mrdA* **=** Δ*PBP2*) in which a *STM1910* (*PBP2SAL*)::3xFLAG-Kan allele was introduced by generalized transduction using the bacteriophage P22 H105/1 int201 (Schmieger, Molecular General Genetics 1972, 119: 75-88). **Genotype:** Δ*mrdA PBP2SAL*::3xFLAG-Kan.

**MD5545.** Bacterial strain derivate of S. Typhimurium MD5060 (Δ*mrdA PBP2SAL*::3xFLAG-Kan) in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** Δ*mrdA PBP2SAL*::3xFLAG.

**MD5547:** Bacterial strain derivate of S. Typhimurium MD5545 (Δ*mrdA PBP2SAL*::3xFLAG) in which a *STM1836 (PBP3SAL)::3xFLAG-Kan* allele was introduced by generalized transduction using the bacteriophage P22 H105/1 *int201* (Schmieger, Molecular General Genetics 1972, 119: 75-88). **Genotype:** Δ*mrdA PBP2SAL*::3xFLAG *PBP3SAL*::3xFLAG-Kan.

**MD5098:** Bacterial strain derivate of S. Typhimurium MD2576 (Δ*STM1910* = Δ*PBP2SAL*) in which a *STM1836 (PBP3SAL)::3xFLAG-Kan* allele was introduced by generalized transduction using the bacteriophage P22 H105/1 int201 (Schmieger, Molecular General Genetics 1972, 119: 75-88). **Genotype:** Δ*PBP2SAL PBP3SAL*::3xFLAG-Kan.

**MD5099:** Bacterial strain derivate of S. Typhimurium MD2577 (Δ*STM1836* = Δ*PBP3SAL*) in which a *STM1910 (PBP2SAL)::3xFLAG-Kan* allele was introduced by generalized transduction using the bacteriophage P22 H105/1 int201 (Schmieger, Molecular General Genetics 1972, 119: 75-88). **Genotype:** Δ*PBP3SAL PBP2SAL*::3xFLAG-Kan.

**MD4360:** Bacterial strain derivate of S. Typhimurium MD4356 (Δ*ftsI* = Δ*PBP3*) **(Table 1)** in which a *STM1836* (*PBP3SAL*)::3xFLAG-Kan allele was introduced by generalized transduction using the bacteriophage P22 H105/1 int201 (Schmieger, Molecular General Genetics 1972, 119: 75-88). **Genotype**: Δ*ftsI PBP3SAL*::3xFLAG-Kan.

**MD5511.** Bacterial strain derivate of S. Typhimurium MD4360 (Δ*ftsI PBP3SAL*::3xFLAG-Kan) in which in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype**: Δ*ftsI PBP3SAL*::3xFLAG.

**MD5534:** Bacterial strain derivate of S. Typhimurium MD5511 (Δ*mrdA PBP2SAL*::3xFLAG) in which a *STM1910 (PBP2SAL)::3xFLAG-Kan* allele was introduced by generalized transduction using the bacteriophage P22 H105/1 *int201* (Schmieger, Molecular General Genetics 1972, 119: 75-88). **Genotype:** Δ*mrdA PBP3SAL::3xFLAG PBP2SAL*::3xFLAG-Kan.

**MD5541:** Bacterial strain derivate of S. Typhimurium MD5052 (Δ*mrdA*) in which the *PBP2SAL* gene (SEQ ID No. 1) was replaced in its native chromosomal location by the *mrdA* = *PBP2* gene using Lambda Red-mediated recombination (Ellermeier, C. D., et al. Gene. 2002; 290: 153-161). A DNA fragment was amplified by PCR from genomic DNA of wild type SV5015 strain using two pairs of primers designed with: i) 50 bp upstream of coding sequence of *PBP2SAL* gene and first 25 nucleotides from the PBP2 gene coding sequence (primer "Exchange PBP2SAL by PBP2 - 1", SEQ ID NO: 21; Table 2); and, ii) last 50 bp of PBP2 gene coding sequence and first nucleotides of the KanR cassette (primer "Exchange PBP2SAL by PBP2 - 2", SEQ ID NO: 22; Table 2). This PCR product was named 1-2. A second DNA fragment was amplified from the template vector pKD13 **(Table 1)** using primers "Exchange PBP2SAL by PBP2 - 3" (SEQ ID NO: 23) and "Exchange PBP2SAL by PBP2 - 4" (SEQ ID NO: 24) (Table 2). The resulting PCR product, named 3-4, was joined to product 1-2 by overlap PCR using primers "Exchange PBP2SAL by PBP2 - 1" (SEQ ID NO: 21) and "Exchange PBP2SAL by PBP2 - 4" (SEQ ID NO: 24). This product was used to replace the PBP2SAL gene by the engineered PBP2 (coding sequence complete) followed by a KanR cassette using Lambda Red-mediated recombination (Ellermeier, C. D., et al. Gene. 2002; 290: 153-161). **Genotype:** Δ*mrdA* Δ*PBP2SAL pPBP2SAL::mrdA-KanR.*

**MD5544:** Bacterial strain derivate of S. Typhimurium MD5541 (Δ*mrdA* Δ*PBP2SAL pPBP2SAL::mrdA-KanR*) in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** Δ*mrdA* Δ*PBP2SAL pPBP2SAL*::*mrdA.*

**MD5546:** Bacterial strain derivate of S. Typhimurium MD5544 (Δ*mrdA* Δ*PBP2SAL pPBP2SAL::mrdA*) in which a *STM1836 (PBP3SAL)::3xFLAG-Kan* allele was introduced by generalized transduction using the bacteriophage P22 H105/1 *int201* (Schmieger, Molecular General Genetics 1972, 119: 75-88). **Genotype:** Δ*mrdA* Δ*PBP2SAL pPBP2SAL*::*mrdA PBP3SAL*::3xFLAG-Kan.

**MD5538:** Bacterial strain derivate of S. Typhimurium MD4356 (Δ*ftsI*) **(Table 1)** in which the *PBP3SAL* gene (SEQ ID No. 3) was replaced in its native chromosomal location by the *ftsl* = *PBP3* gene using Lambda Red-mediated recombination (Ellermeier, C. D., et al. Gene. 2002; 290: 153-161). A DNA fragment was amplified by PCR from genomic DNA of wild-type SV5015 strain using two pairs of primers designed with: i) 50 bp upstream of *PBP3SAL* gene and first 25 nucleotides from the *ftsI* gene coding sequence (primer "Exchange PBP3SAL by PBP3 - 1", SEQ ID NO: 17; Table 2); and, ii) last 50 bp of PBP2 gene coding sequence and first nucleotides of the KanR cassette (primer "Exchange PBP3SAL by PBP3 - 2", SEQ ID NO: 18; Table 2). This PCR product was named 1-2. A second DNA fragment was amplified from the template vector pKD13 **(Table 1)** using primers "Exchange PBP3SAL by PBP3 - 3" (SEQ ID NO: 19) and "Exchange PBP3SAL by PBP3 - 4" (SEQ ID NO: 20) (Table 2). The resulting PCR product, named 3-4, was joined to product 1-2 by overlap PCR using primers "Exchange PBP3SAL by PBP3 - 1" (SEQ ID NO: 17) and "Exchange PBP3SAL by PBP3 - 4" (SEQ ID NO: 20). This product was used to replace the *PBP3SAL* gene by the engineered *ftsI* (coding sequence complete) followed by a KanR cassette using Lambda Red-mediated recombination (Ellermeier, C. D., et al. Gene. 2002; 290: 153-161). **Genotype:** Δ*ftsI* Δ*PBP3SAL pPBP3SAL::ftsI-KanR.*

**MD5542:** Bacterial strain derivate of S. Typhimurium MD5538 (Δ*ftsI* Δ*PBP3SAL pPBP3SAL::ftsI-KanR*) in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** Δ*ftsI* Δ*PBP3SAL pPBP3SAL*::*ftsI*

**MD5543:** Bacterial strain derivate of S. Typhimurium MD5542 (Δ*ftsI* Δ*PBP3SAL pPBP3SAL::ftsI*) in which a *STM1910 (PBP2SAL)::3xFLAG-Kan* allele was introduced by generalized transduction using the bacteriophage P22 H105/1 *int201* (Schmieger, Molecular General Genetics 1972, 119: 75-88). **Genotype:** Δ*ftsI* Δ*PBP3SAL pPBP3SAL*::*ftsI PBP2SAL*::3xFLAG-Kan

**MD5549:** Bacterial strain derivate of S. Typhimurium MD5052 (Δ*mrdA*) transformed with plasmid pAC-6xHis-STM1910 (= 6xHis-PBP2SAL). To construct pAC::PBP2SAL, STM1910 (*PBP2SAL* or *SpeI*) fragment was ligated into pAC-Plac (Lobato-Marquez, D., et al. Sci Rep. 2015; 5: 9374).

**MD5550:** Bacterial strain derivate of S. Typhimurium MD5052 (Δ*mrdA*) transformed with plasmid pAC-6xHis-STM1910 (= 6xHis-PBP2SAL-S326A). To construct the pAC-HIS::PBP2SAL-S326A variant, a *STM1910 (PBP2SAL)* allele encoding the transpeptidase point mutation S326A (substitution of GCA by TCA at bases 976-978) was generated. Two fragments of DNA were amplified from genomic DNA of wild-type strain SV5015 using primers fwSpeI-PBP2*/PBP2SAL S326A RV (SEQ ID NO: 27/SEQ ID NO: 16) and PBP2SAL S326A FW/ revSpePBP2* (SEQ ID NO: 15/SEQ ID NO: 28) **(Table 2)**. Overlap PCR was performed with primers fwSpeI-PBP2*/ revSpeI-PBP2* (SEQ ID NO: 27/SEQ ID NO:28) **(Table 2)** and the full fragment was digested with Spel and cloned into pAC (Lobato-Marquez, D., et al. Sci Rep. 2015; 5: 9374). The final construct was sequenced to confirm the point mutation and to ensure no undesired mutation.

**MD2840.** Bacterial strain derivate of S. Typhimurium SV5015 following transformation with plasmid pKD46 **(Table 1).**

**MD5061:** Bacterial strain derivate of S. Typhimurium MD4356 (Δ*ftsI*) in which a Δ*mrdA::KanR* allele obtained from strain MD5049 (Δ*mrdA::KanR*) was introduced by generalized transduction using the bacteriophage P22 H105/1 *int201* (Schmieger, Molecular General Genetics 1972, 119: 75-88). **Genotype:** Δ*ftsI ΔmrdA::KanR*

**MD5063:** Bacterial strain derivate of S. Typhimurium MD5061 (Δ*ftsI ΔmrdA::KanR*) in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** Δ*ftsI* Δ*mrdA =* Δ*PBP2* Δ*PBP3.*

**MD5508:** Bacterial strain derivate of E. coli RP41 **(Table 1)** that was transformed with plasmid pAC-HIS::PBP3Citrobacter. The PBP3SAL gene of *C*. *freundii* (SEQ ID No. 9) was amplified from genomic DNA of this bacterium using the primers fwSpel-PBP3Citro (SEQ ID NO: 35) and revPvul-PBP3Citro (SEQ ID NO: 36) **(Table 2)** and cloned in plasmid pAC-HIS **(Table 1)** using Spel and Pvul restriction enzymes. **Genotype:** RP41 pAC-HIS::PBP3-*Citrobacter.*

**MD2580.** Bacterial strain derivate of S. Typhimurium MD2576 (Δ*STM1910* = Δ*PBP2SAL*) in which the Δ*STM1836*-Kan allele from donor strain MD2502 **(Table 1)** was introduced by generalized transduction using the bacteriophage P22 H105/1 *int201* (Schmieger, Molecular General Genetics 1972, 119: 75-88). **Genotype**: Δ*PBP2SAL* Δ*PBP3SAL*-Kan

**MD2588.** Bacterial strain derivate of S. Typhimurium MD2580 (Δ*PBP2SAL* Δ*PBP3SAL*-Kan) in which in which the KanR cassette was excised from the chromosome with the excisionase enzyme encoded in the plasmid pCP20 following the method of Datensko and Wanner (Proc Natl Acad Sci U S A. 2000; 97: 6640-6645). **Genotype:** Δ*PBP2SAL* Δ*PBP3SAL.*

### Bacterial growth

*E.coli* and *S*. Typhimurium strains were grown in Luria-Bertani (LB) broth (1% casein peptone, 0.5% yeast extract, 0.5% sodium chloride; 75852, Affymetrix) or phosphate-carbon-nitrogen (PCN) minimal medium. When necessary, pH was buffered with 80 mM MES [2-(N-morpholino) ethanesulfonic acid] (69892, Sigma-Aldrich). The composition of PCN medium was: 4 mM Tricine [N-[Tris(hydroxymethyl)methyl]glycine], 0.1 mM FeCl3, 376 µM K2SO4, 50 mM NaCl, 15mM NH4Cl, 1 mM MgSO4, 1 µM CaCl2, 0.4% (w/v) glucose, 0.4 mM inorganic phosphate (Pi), and micronutrients. 80 mM MES adjusted to the desired value with NaOH was used to buffer the medium. The antibiotic concentrations used to grow plasmid-harboring strains were: ampicillin, 100 µg/ml; chloramphenicol, 10 µg/ml; and kanamycin 30 µg/ml for LB 7.0 and 60 µg/ml for acidic LB (pH 4.6).

### Spot dilution assay and antibiotic resistance assays.

PCN plates for spot dilution assays were prepared by mixing 7.5 ml of sterile PCN medium (2x concentrated) at the desired pH and 7.5 ml of autoclaved agar (30 mg/mL). The mixture was supplemented with 0.01% (w/v) yeast extract (YE). Cultures were grown in PCN pH 4.6 medium overnight and adjusted to same optical density values. These cultures were serially 10-fold diluted. 5 µl of each dilution was spotted onto PCN plates with or without antibiotic added.

Antibiotic susceptibility was determined using MIC strips (MIC Test Strip; Liofilchem) in PCN agar plates containing 0.01% YE at neutral or acid pH 4.6. S. Typhimurium cultures grown in PCN 4.6 were normalized to an optical density at 600 nm (OD600) of 0.1 and diluted 1:10. One hundred twenty microliters of this dilution were spread onto PCN plates using glass beads. The assays were repeated at least in three independent biological replicates.

### PBP synthesis during a shift from pH 7.4 to 4.6.

*S*. *Typhimurium* strain harbouring the PBP2SAL::3xFlag PBP3SAL::3xFlag tagged alleles was incubated overnight at 37°C in liquid PCN 7.4 medium. Cultures were then prepared to a starting OD600 of 0.05 in PCN 4.6 and grown in parallel in distinct flasks at 37°C with 150 rpm under shaking conditions. At different times, bacteria from a flask were collected for protein analysis by western blot. A sample-independent flask was used in parallel in order to measure culture optical density (OD600) over time. The experiment was repeated two times.

### Macrophage infection and colony-forming unit counting.

The murine macrophage line RAW 264.7 (400319, Cell Lines Service, CLS, https://clsgmbh.de/) was cultured in Dulbecco's modified Eagle's medium (DMEM) with 5% fetal bovine serum (FBS) and 4 mM glutamine in an incubator at 37°C in a 5% CO2 atmosphere. RAW 264.7 macrophages were seeded in 24-well plates (353047, Falcon) at a density of 2x10⁵ cells/well and pre-cultured for 24 h before infection. Macrophages were activated with 2.5 ng/ml interferon-γ (315-05, Peprotech) 24 h prior infection. Culture medium was replaced by fresh medium without interferon-γ and infected with S. Typhimurium strains at a multiplicity of infection (MOI) of 10:1 during 10 min. Macrophages were washed with PBS supplemented with 0.5 mM MgCl2 and 0.9 mM CaCl2 (complete PBS) and extracellular non-phagocytosed bacteria were killed by incubation in medium containing gentamicin (100 µg/ml for 1 h, followed by 10 µg/ml for the remaining incubation). If indicated, aztreonam (ATM) (10 µg/mL) was added at 1 hpi. Infected macrophages were lysed at 1 and 8 hpi. Colony-forming units (CFU) were determined after two washes of the culture with complete PBS buffer, lysis with sterile solution containing 1% (w/v) Triton X 100 and 0.1% (w/v) sodium dodecyl sulfate (SDS) in PBS and plating on LB pH 7.0 or LB pH 4.6. The assays were repeated at least in three independent biological replicates.

### Phase contrast microscopy.

Bacteria were scraped from PCN plates of spot dilution assays and streaked in 500µl PBS buffer. After centrifugation at 4,300 x g for 2 min, bacteria were fixed with 3% (w/v) paraformaldehyde (PFA) for 15 min at room temperature and then the sample was diluted to 1% PFA with PBS. Images were acquired on an inverted Leica DMI 6000B microscope with an automated CTR/7000 HS controller (Leica Microsystems) and an Orca-R2 charge-coupled-device (CCD) camera (Hamamatsu Photonics). The assays were repeated at least in three independent biological replicates.

### Membrane fractions and Bocillin-FL binding assay for PBP2SAL (SEQ ID NO: 2).

Membranes were isolated from 100 ml cultures of *E. coli* SP4500 [*mrdA* (ts)] (Garcia del Portillo, F. & de Pedro, M. A. J Bacteriol. 1990; 172: 5863-5870) strain harbouring empty vector or vectors expressing S. Typhimurium PBP2 or PBP2SAL in LB at 30°C. At OD600 = 0.3, 0.2% (w/v) arabinose was added to induce PBP2 and PBP2SAL expression. After 2 h induction, bacteria were pelleted by centrifugation (4,400 x g, 10 min, 4°C), washed in 0.1 M phosphate buffer adjusted to pH 7.4, 5.8 or 4.6 and frozen at -80°C. Preparation of lysates and binding assay with Bocillin were done as previously described (Castanheira, S. et al. MBio. 2017; 19;8(6): e01685-17). The assays were repeated at least in three independent biological replicates.

### BALB/c mice experiments.

Groups of three 8-week-old female BALB/c mice were inoculated with the following isogenic strains: MD5064 (PBP2SAL::3xFlag PBP3SAL::3xFlag), MD5063 (ΔPBP2, ΔPBP2SAL), MD5544 (ΔPBP2 Δ*PBP2SAL pPBP2SAL*::PBP2), MD5542 (ΔPBP3, Δ*PBP3SAL* and ΔPBP3 Δ*PBP3SAL pPBP3SAL*::PBP3). The doses used were 2x10³ CFU in 200 µl of sterile PBS. Along four days, mice received intraperitoneally every 24 h a regime consisting in 10 mg mecillinam (MEC) or 2.5 mg aztreonam (ATM) per animal in 200 µl of sterile water. Mice were sacrificed four days post inoculation and spleens homogenized in 5 ml of sterile PBS. Aliquots (100 µl) of 10-fold serial dilutions of the homogenates were cultured in LB pH 4.6 plates to quantify the number of viable S. Typhimurium. To detect PBPs *in vivo,* spleen extracts from three mice were prepared at 72 hpi as described previously (Dominguez-Bernal, G. et al. Mol Microbiol. 2004; 53: 1437-1449). The protocols used in the study were approved by the Consejería de Medio Ambiente de la Comunidad de Madrid (ref. PROEX 110/19).

### Immunoblot analyses.

The following antibodies were used as primary antibodies: mouse monoclonal anti-Flag (1:5,000; F1804, Sigma), mouse monoclonal anti-HA (1:2,000), rabbit polyclonal anti-PBP2 (1:1,000; our lab collection), rabbit polyclonal anti-PBP34 (1:1,000). Goat polyclonal anti-mouse (1:20,000) or anti-rabbit IgG (1:20,000) conjugated to horseradish peroxidase (catalog no. 1706516 and 176515, respectively; Bio-Rad) were used as secondary antibodies. Proteins were separated by 4-20% precasted polyacrylamide gels (4561096, Bio-Rad), 7% tricine gels, or 6% acrylamide gels.

### PCR reactions

PCR was performed using Q5 polymerase (NEB) according to the manufacturer's instructions. PCR fragments were purified using the Speedtools PCR Clean-Up kit (21.202, Biotools).

### Statistical analysis.

Data were analyzed with GraphPad Prism, version 5.0, software (GraphPad Inc. San Diego, CA) using a Student *t* test with Mann-Whitney non-parametric post-test. *P* value lower than 0.05 was considered significant. Experiments were repeated as a minimum of three independent biological replicates.

### EXAMPLE 1. An expanded repertoire of peptidoglycan enzymes in S. Typhimurium.

As in the rest of *Salmonella* enterica serovars of subspecies I, II, III, IVa, IVb and V, the genome of serovar Typhimurium (S. Typhimurium) encodes a set of enzymes predicted to be involved in PG metabolism that are absent in *E. coli,* its closest phylogenetic relative. Among these are a D,L-endopeptidase induced by intracellular bacteria that cleaves the D-glutamic (D-Glu)-*meso*-diaminopimelic acid (meso-Dap) bond in the stem peptide of the peptidoglycan, named EcgA (Rico-Perez et al., Molecular Microbiology. 2016; 99: 546-556); and two predicted PBPs of size 623 and 581 amino acids, which we named PBP2SAL (SEQ ID NO: 2) and PBP3SAL (SEQ ID NO: 4), respectively.

PBP2SAL (SEQ ID NO: 2) and PBP3SAL (SEQ ID NO: 4) have 64% and 63% amino acid identity to PBP2 (SEQ ID NO: 6) and PBP3 (SEQ ID NO: 8), involved in cell elongation and division, respectively. Consistent with a role in peptidoglycan metabolism, PBP2SAL (SEQ ID NO: 2) and PBP3SAL (SEQ ID NO: 4) bear a transpeptidase functional domain (Pfam:PF00905) and conserve the residues critical for catalysis **(****Fig. 2****).**

Importantly, PBP2 (SEQ ID NO: 6) and PBP3 (SEQ ID NO: 8) inhibition by beta-lactams results in shape alterations and cell death in Gram-negative bacteria. BLAST analyses reveal that PBP2SAL (SEQ ID NO: 2) and PBP3SAL (SEQ ID NO: 4) are also present in opportunistic bacterial pathogens of environmental origin **(****Fig. 3****).** In *S*. *Typhimurium,* PBP3SAL (SEQ ID NO: 4) expression responds to a phagosomal environmental cue like acid pH and promotes pathogen division inside eukaryotic cells (**Fig. 5**). Given the clinical evidence showing that beta-lactams are ineffective to eradicate intracellular S. Typhimurium leading to frequent relapses (more than 50% cases in some paediatric studies), the inventors state that PBP2SAL (SEQ ID NO: 2) and PBP3SAL (SEQ ID NO: 4) as enzymes that facilitate pathogen adaptation to the phagosomal niche, conferring antibiotic resistance in this hostile environment.

To test this hypothesis, we monitored PBP2SAL (SEQ ID NO: 2) and PBP3SAL (SEQ ID NO: 4) relative levels in different growth conditions using the strain MD5064 (see material and methods), expressing from their native chromosomal locations PBP2SAL::3xFlag and PBP3SAL::3xFlag variants tagged in their C-termini. PBP2 (SEQ ID NO: 6) and PBP3 (SEQ ID NO: 8) levels were determined in the same strain using anti-PBP2 and anti-PBP3 antibodies.

PBP2SAL and PBP3SAL 3xFLAG-tagged-variants retain function as they promote cell elongation and division in the absence of PBP2 and PBP3 **(****Fig.4A****)** (Castanheira et al. 2017, mBio 8 10.1128/mBio.01685-17). Using this doubly-tagged strain, the inventors registered high *de novo* expression of PBP2SAL and PBP3SAL in intracellular bacteria collected from macrophages **(****Fig. 5A****)**, main host cell type in which S. Typhimurium persists, proliferates and disseminates to distal systemic sites. Inside macrophages, PBP2SAL and PBP3SAL expression prevailed over that of PBP2 and PBP3, these later detected at similar levels as in extracellular bacteria **(****Fig. 5A****).** PBP2SAL and PBP3SAL prevalence was also observed *in vitro* in bacteria growing in a minimal acidified medium (pH 4.6) with limited amounts of nutrients **(****Fig. 1****)**, conditions that mimic the intra-phagosomal environment. Acid pH and nutrient limitation are, therefore, both required to switch to preferred PBP2SAL and PBP3SAL synthesis. Thus, growth of *S*. Typhimurium in the Mueller-Hinton nutrient-rich media used in standard antimicrobial assays does not recapitulate prevalence of PBP2SAL and PBP3SAL over PBP2 and PBP3, even in acidic conditions **(****Fig. 6****).**

Based on these results, the inventors sought to determine the level of expression of PBP2SAL and PBP3SAL *in vivo.* Unexpectedly, we observed that the substitution of PBP2 and PBP3 by PBP2SAL and PBP3SAL was complete *in vivo* following *S*. Typhimurium colonization of spleen in BALB/c mice **(****Fig. 5B****).** These results indicated that PBP2SAL and PBP3SAL, which are produced by intracellular *S*. Typhimurium, are the PBPs driving cell elongation and division *in vivo* inside the phagosomal compartment.

### EXAMPLE 2. An acidified environment limited in nutrients decreases S. Typhimurium susceptibility to beta-lactams.

PBP2SAL and PBP3SAL are produced *de novo* by intra-phagosomal *S*. Typhimurium and the infections caused by this pathogen are intracellular. Thus, the inventors hypothesised that the failure of beta-lactams in eradicating salmonellosis could be related to these two enzymes. Specifically, our hypothesis is that a reduction in the affinity of PBP2SAL and PBP3SAL for beta-lactams would drive antibiotic resistance.

To analyse this, antibiotic susceptibility was tested in *in vitro* conditions favouring production of either PBP2/PBP3 (neutral pH) or PBP2SAL/PBP3SAL (acid pH) **(****Fig. 7****).** We used beta-lactams widely used in the clinics that bind with high affinity to PBP2 (mecillinam, MEC) or PBP3 (the monobactam aztreonam, ATM). MEC and ATM inhibit bacterial growth at pH 7.4 (condition in which PBP2 and PBP3 are expressed) but not at pH 4.6, condition in which PBP2SAL and PBP3SAL expression is favoured **(****Fig. 7A****).** Several observations discarded diminished activity of the antibiotics in acid pH as basis of ATM and MEC resistance. First, PBP3SAL, which is expressed in acid pH **(****Figs. 1****,** **5****)**, has lower affinity than PBP3 for beta-lactams and it losses completely the ability to bind the antibiotic at neutral pH (Castanheira et al. 2017, mBio 8 10.1128/mBio.01685-17). Second, affinity binding tests with the fluorescent penicillin marker Bocillin-FL confirmed that PBP2SAL has also lesser affinity for beta-lactams compared to PBP2 **(****Fig. 8****).** Taken together, these results indicated that S. Typhimurium adaptation to the hostile acidic phagosomal niche is accompanied by expression of two PBPs that display reduced affinity for widely used beta-lactam antibiotics targeting PBP2 and PBP3.

### EXAMPLE 3. PBP3SAL confers resistance to beta-lactams in in vitro conditions

To determine whether PBP2SAL and PBP3SAL contribute to the mechanism of antibiotic resistance observed in acid pH, a first series of isogenic mutants lacking PBP2, PBP2SAL or expressing PBP2 under the acid pH-responding promoter of the gene encoding PBP2SAL were generated **(****Fig. 10****).** This later strain allowed us to test at acid pH the susceptibility to antibiotics binding with high affinity to PBP2 in the absence of PBP2SAL and, as consequence, to prove whether the antibiotic retains activity at that pH. The lack of either PBP2SAL or PBP2 does not alter susceptibility to MEC at pH 4.6 in these *in vitro* conditions **(****Fig. 10****).** This behaviour correlates to remnant amounts of PBP2 observed in bacteria growing *in vitro* at pH 4.6 **(****Fig. 1****)**, which impeded a stage of complete substitution of PBP2 by PBP2SAL **(****Fig. 10****).** Exclusive expression of one of these enzymes is however reached in neutral pH 7.4 (no PBP2SAL detected) in which the mutant lacking PBP2 is resistant to MEC **(****Fig. 10****).** Thus, the presence of PBP2 at pH 4.6 in bacteria growing *in vitro* in a medium with limited nutrients made not possible the assessment of an antibiotic resistance phenotype linked to PBP2SAL.

Furthermore, isogenic mutants lacking either PBP3 or PBP3SAL and a third strain devoid of PBP3SAL that expresses PBP3 in response to acid pH was also generated **(****Fig. 7****).** This later strain allowed us to test efficacy at acid pH of antibiotics binding with high affinity to PBP3. All strains used (wild-type and derivates) are susceptible to ATM at neutral pH (7.4) whereas only those expressing PBP3SAL are resistant at pH 4.6 **(****Fig. 7B****).** This result demonstrated that PBP3SAL is the primary cause of ATM resistance in an acidified environment having low amount of nutrients. Likewise, these findings discarded significant reduction of ATM activity at pH 4.6 since the antibiotic showed antimicrobial activity at this pH against mutants lacking PBP3SAL **(****Fig. 7B****).**

As ATM resistance mediated by PBP3SAL at pH 4.6 is easily detected *in vitro* **(****Fig. 7B****)**, the minimal inhibitory concentration (MIC) values to ATM in bacteria lacking PBP3 or PBP3SAL were estimated. The sole switch in pH value from 7.4 to 4.6 results in a notorious increase in the MIC value in *wild-type* bacteria (<0.016 µg/mL to 0.125 µg/mL) **(****Fig. 11A****).** Noteworthy, the MIC value further increases at pH 4.6 in bacteria lacking PBP3 (MIC = 0.75 µg/mL) **(****Fig. 11A****).** This finding indicated that wild-type bacteria may still express minor, albeit undetectable, levels of PBP3 when growing *in vitro* at pH 4.6. Exposure of *S*. Typhimurium to other beta-lactams known to bind with high affinity to PBP3 like cefotaxime (CTX), ceftazidime (CAZ), cephalotin (KF) and cefuroxime (CXM) showed that PBP3SAL expression following the switch to acid pH (4.6) results in decreased susceptibility to these drugs.

Taken together, these results demonstrated that PBP3SAL, which is induced by *S*. Typhimurium inside macrophages and in mouse target organs **(****Fig. 5****)**, affects antibiotic susceptibility to frequently-used cephalosporins and monobactams.

### EXAMPLE 4. S. Typhimurium exhibits beta-lactam resistance inside eukaryotic cells

Intracellular *S*. *Typhimurium* resides mostly within acidic phagosomes. As PBP3SAL promotes ATM resistance *in vitro* **(****Fig. 7****)** and its production is stimulated by bacteria inside macrophages **(****Fig. 5A****)**, the inventors reasoned that S. Typhimurium should be intrinsically less susceptible to this beta-lactam inside eukaryotic cells.

ATM was added at 10 µg/mL to macrophage cultures previously infected with *S*. Typhimurium, a concentration that was adjusted to ensure decreased viability of intracellular wild-type bacteria. In support to our hypothesis, the lack of PBP3SAL render intra-phagosomal bacteria more susceptible to ATM than those bacteria lacking PBP3 **(****Fig. 12A****).** These results indicate that the *de novo* appearance of PBP3SAL as part of the adaptive *S*. *Typhimurium* response to the intracellular niche has a strong impact on antibiotic susceptibility.

### EXAMPLE 5. PBP2SAL and PBP3SAL reduce effectiveness of beta-lactams for eradicating S. Typhimurium in vivo

The present invention shows that PBP2SAL and PBP3SAL replace PBP2 and PBP3 *in vivo* in BALB/c mice **(****Fig. 5****).** Given that PBP2SAL and PBP3SAL bind beta-lactams with less affinity than PBP2 and PBP3, the inventors hypothesized that S. Typhimurium should be innately less susceptible to these drugs when colonizing mouse organs.

To test this hypothesis, ATM or MEC were administrated to BALB/c mice challenged with S. Typhimurium isogenic mutants lacking PBP2, PBP3, PBP2SAL or PBP3SAL. Antibiotic treatment did not result in a statistically significant decrease the load of wild-type bacteria in spleen although a slightly effect of reduced viability was seen in the case of MEC **(****Fig. 12B****).** Consistently to our hypothesis, mice challenged with bacteria lacking PBP2SAL or PBP3SAL displayed however a significant decrease of bacterial loads in response to MEC and ATM, respectively **(****Fig. 12B****).** This result showed that PBP2SAL and PBP3SAL mediate antibiotic resistance *in vivo.* In support to this conclusion, MEC and ATM have lesser effect in mice challenged with mutants lacking PBP2 or PBP3 **(****Fig. 12B****).** Furthermore, artificial expression of PBP2 or PBP3 from the acid-responsive PBP2SAL and PBP3SAL promoters resulted in exacerbated antibiotic susceptibility **(****Fig. 12B****).**

In sum, these data proved that the two novel PBPs, PBP2SAL (SEQ ID NO: 2) and PBP3SAL (SEQ ID NO: 4), that *S*. Typhimurium produces *in vivo* in response to intracellular host cues confer antibiotic resistance in the absence of a drug-mediated selective pressure.

### EXAMPLE 6. The PBP3SAL ortholog of Citrobacter freundii has also evolved to function at acidic pH.

Similar to PBP3SAL of S. Typhimurium, the PBP3SAL ortholog protein of *C*. *freundii* (SEQ ID NO. 10), restores cell division only in acidic pH in *E. coli* strain RP41, which is unable to divide at 42°C (**Fig. 14**)**.** By contrast, PBP3 of S. Typhimurium restores cell division at both neutral and acidic pH (**Fig. 14**)**.** This result reinforces the idea that additional PBPs like PBP3SAL evolved to perform specialized functions in acidic environments.

### EXAMPLE 7. Method to select inhibitors of PBP2SAL and/or PBP3SAL, induced by Salmonella inside eukaryotic cells.

The mutant MD5063 is defective for both PBP2 and PBP3. Likewise, PBP2SAL and PBP3SAL are absolutely required for bacterial viability in acid pH in this mutant. An assay in which this double mutant [*S*. *enterica* serovar Typhimurium strain MD5063 (ΔPBP2, ΔPBP3)] will be screened in parallel with wild-type bacteria, *S*. *enterica* serovar Typhimurium strain SV5015, was generated.

The results of the spot assay are shown in **Fig. 13A** for two hypothetical inhibitors, one binding with low affinity and the other with high affinity to PBP2SAL and/or PBP3SAL (**Fig. 13B**).

The assay must be previously designed based in the use of multi-well plates in which the chemical library is screening in both neutral and acid pH and in wild-type and the double mutant ΔPBP2 ΔPBP3 **(****Fig. 13A****).** Once positives as potential specific inhibitors of PBP2SAL and/or PBP3SAL are found **(****Fig. 13B****)**, these should be tested individually in plates and, in addition, in standard assays to determine minimal inhibitory concentrations (MIC).

To assess whether the inhibitor identified in the screening shown in **Fig. 13** is specific of PBP2SAL or PBP3SAL, the same principle (two pH values and comparison with wild-type strain as shown in **Figs. 13A and 13B**) can be used but now using single ΔPBP2 and ΔPBP3 mutants (named as MD4356 and MD5052 strains, respectively).

If the inhibitor is specific of PBP2SAL, there is growth of the ΔPBP3 mutant in acid pH, but there is no growth of the ΔPBP2 mutant in acid pH. However, if the inhibitor is specific of PBP3SAL, there is growth of the ΔPBP2 mutant in acid pH but there is no growth of the ΔPBP3 mutant in acid pH.

## Claims

1. An INDAR inhibitor for use as medicament, preferably an antibiotic.

2. An INDAR inhibitor for use in the treatment and/or prevention of intracellular bacterial infections.

3. An INDAR inhibitor for use according to any of claims 1 or 2 wherein the INDAR inhibitors recognize a PBP protein selected from the list consisting of: a PBP protein having at least 80% identity with SEQ ID NO: 2 and/or a PBP protein having at least 80% identity with SEQ ID NO: 4, preferably recognize a PBP protein comprises the sequences as set forth in SEQ ID NO: 2 and/or SEQ ID NO: 4.

4. An INDAR inhibitor for use according to any of claims 1 or 3 wherein the bacteria are selected from the list consisting of: *Salmonella spp., Citrobacter* spp., *Enterobacter* spp., *Klebsiella* spp., *Leciercia spp., Lelliottia* spp., *Cedecea* spp., *Raoultella* spp., *Kluyvera* spp,, *Pantoea* spp, *Erwinia* spp., *Mixta* spp., *Serratia* spp., *Escherichia* spp., *Pluribacter* spp., *Rosenbergiella* spp., *Morganella* spp., *Tatumella* spp., *Acinetobacter* spp., and *Haemophilus* spp.

5. An INDAR inhibitor for use according to claim 4 wherein the bacteria are selected from the list consisting of: *Salmonella spp.,* preferably the species selected from the list consisting of: *S*. *enterica and S. bongori,* and *Citrobacter* spp. preferably the species selected from the list consisting of: *C. koseri, C. freundii, C. youngae, C. braakii, C. werkmanii, C. murliniae, C. gillenii, C. sedlakii, C. pasteurii, C. eropaeus, C. amalonaticus, C. farmeri, C. rodentium* and *C. portucalensis.*

6. Method *in vitro* to identify INDAR inhibitors wherein the method comprises:
a) Contacting a candidate compound with a bacterial culture of pathogenic bacteria, wherein the pathogenic bacteria comprises a non-functional gene encoding for a PBP protein selected from the list consisting of: a PBP protein having at least 80% identity with SEQ ID NO: 6 and/or a PBP protein having at least 80% identity with SEQ ID NO: 8;
b) Incubate the candidate compound with the bacterial culture of step a) in an acidic pH range from 4.0 to 5.0;
c) Comparing the growth of bacterial culture of step b) with the growth of the corresponding pathogenic wild-type bacterial culture in the presence of the candidate compound;
wherein a decreased growth of the pathogenic bacterial culture comprises the non-functional gene encoding for a PBP protein selected from the list consisting of: a PBP protein having at least 80% identity with SEQ ID NO: 6 and/or a PBP protein having at least 80% identity with SEQ ID NO: 8, regarding the growth of corresponding pathogenic wild-type bacterial culture, indicates that the candidate compound is an INDAR inhibitor.

7. Method according to claim 6 wherein the non-functional gene encoding for a PBP protein comprises the sequences as set forth in SEQ ID NO: 6 and/or SEQ ID NO: 8.

8. Method according to any of claims 6 to 7 wherein the pathogenic bacteria are selected from the list consisting of: *Salmonella spp., Citrobacter* spp., *Enterobacter* spp., *Klebsiella* spp., *Leciercia spp., Lelliottia* spp., *Cedecea* spp., *Raoultella* spp., *Kluyvera* spp,, *Pantoea* spp, *Erwinia* spp., *Mixta* spp., *Serratia* spp., *Escherichia* spp., *Pluribacter* spp., *Rosenbergiella* spp., *Morganella* spp., *Tatumella* spp., *Acinetobacter* spp., and *Haemophilus* spp.

9. Method according to claim 8 wherein the pathogenic bacteria are selected from the list consisting of: *Salmonella spp.,* preferably the species selected from the list consisting of: *S*. *enterica and S. bongori,* and *Citrobacter* spp. preferably the species selected from the list consisting of: *C. koseri, C. freundii, C. youngae, C. braakii, C. werkmanii, C. murliniae, C. gillenii, C. sedlakii, C. pasteurii, C. eropaeus, C. amalonaticus, C. farmeri, C. rodentium* and *C. portucalensis.*

10. Method according to any of claims 6 to 9 wherein the acidic pH ranges from 4.3 to 4.8, preferably 4.6.

11. Method according to any of claim 6 to 10 wherein the INDAR inhibitor recognize a PBP protein having at least 80% identity with SEQ ID NO: 2 and/or a PBP protein having at least 80% identity with SEQ ID NO: 4, preferably, the sequences as set forth in SEQ ID NO: 2 and/or SEQ ID NO: 4.

12. An INDAR inhibitor obtainable from the method as defined in any of the claims 6 to 11.

13. Use of a pathogenic bacterial cell comprising a non-functional gene encoding for a PBP for the *in vitro* identification and/or validation of INDAR inhibitors.

14. A *Salmonella* Typhimurium mutant cell **characterized in that** does not express at least one polynucleotide sequence encoding at least one polypeptide selected from the list consisting of: PBP2 (SEQ ID NO: 6), PBP2SAL (SEQ ID NO: 2), PBP3 (SEQ ID NO: 8), PBP3SAL (SEQ ID NO: 4) or any combinations thereof.

15. A *Salmonella* Typhimurium mutant host cell according to claim 14 wherein the mutant does not express the polynucleotide sequence encoding the PBP2 polypeptide (SEQ ID NO: 6); or the mutant does not express the polynucleotide sequence encoding the PBP2SAL polypeptide (SEQ ID NO: 2), or the mutant does not express the polynucleotide sequence encoding the PBP2 polypeptide (SEQ ID NO: 6) and the PBP3 polypeptide (SEQ ID NO: 8), or the mutant has been replaced the PBP2SAL gene (SEQ ID NO. 1) in its native chromosomal location by the PBP2 gene (SEQ ID NO: 5), or the mutant has been replaced the PBP3SAL gene (SEQ ID NO. 3) in its native chromosomal location by the PBP3 gene (SEQ ID NO: 7), or the mutant does not express the polynucleotide sequence encoding the PBP2SAL polypeptide (SEQ ID NO: 2) and the polynucleotide sequence encoding the PBP3SAL polypeptide (SEQ ID NO: 4), preferably the mutant does not express the polynucleotide sequence encoding the PBP2 polypeptide (SEQ ID NO: 6) and the PBP3 polypeptide (SEQ ID NO: 8) or the mutant does not express the polynucleotide sequence encoding the PBP2 polypeptide (SEQ ID NO: 6).
